# EUROPEAN PATENT APPLICATION

(11) **EP 0 879 554 A1**
(43) Date of publication of application: **25.11.1998**
(21) Application number: 96941871.4
(22) Date of filing: 13.12.1996
(51) Int. Cl.: A01H 5/00, C12N 15/29

(54) **MOLD-RESISTANT PLANT AND PROCESS FOR PRODUCING THE SAME**

(30) Priority: 15.12.1995 JP 347823/95
(71) Applicant: KIRIN BEER KABUSHIKI KAISHA, Chuo-Ku, Tokyo 104 (JP)
(72) Inventor: KAKITANI, Makoto;-Kirin Beer K. K. K. Gij. Kenk., Kanagawa 236 (JP); UMEMOTO, Naoyuki-Kirin Beer K. K. K. Giju. Kenky., Kanagawa 236 (JP); ISHIDA, Isao-Kirin Beer Kab.Kaisha K. Giju. Kenk., Kanagawa 236 (JP); YAMAOKA, Naoto, Hokkaido 062 (JP)
(74) Representative: Kolb, Helga, Dr. Dipl.-Chem.
(86) International application number: JP9603653
(87) International publication number: WO9722242

(57) **Abstract**

A method for creating a plant having resistance to pathogenic fungi comprising incorporating a DNA sequence coding for a glucan elicitor receptor into a chromosome of a plant and expressing the glucan elicitor receptor in the plant; a plant having resistance to pathogenic fungi, the plant having a DNA sequence coding for a glucan elicitor receptor transferred thereinto and expressing the glucan elicitor receptor; and uses of the DNA sequence.

## Description

### TECHNICAL FIELD

The present invention relates to fungus-resistant plants and a method for creating such plants. More specifically, the present invention relates to fungus-resistant plants into which a DNA sequence coding for a glucan elicitor receptor has been transferred and a method for creating such plants.

### BACKGROUND ART

It is known that plants synthesize and accumulate an antibiotic agent called phytoalexin in response to infection with pathogens (M. Yoshikawa (1978) Nature 257: 546). Some plant pathogens were found to have the substances that induce them to perform such a resistance reaction (N.T. Keen (1975) Science 187: 74), which are called "elicitors". The biochemical process from the infection of plants with pathogens to the synthesis and accumulation of phytoalexin is believed to be as follows:

When the mycelium of a pathogen invades a plant cell, glucanase in the plant cell works so as to cleave polysaccharides on the surface of the pathogen mycelial wall, thereby liberating an elicitor. If the elicitor binds to a receptor in the plant cell, a second messenger which plays a role in signal transduction is produced. The signal transduction substance is incorporated in the nucleus of the plant cell and activates the transcription of the genes coding for phytoalexin synthesize enzymes to induce a phytoalexin synthesis. At the same time, the phytoalexin degradation is inhibited. As a result, phytoalexin is efficiently accumulated in the plant cell.

A phytoalexin playing an important role in the resistance of soybean is called glyceollion and its structure has been determined (M. Yoshikawa et al. (1978) Physiol. Plant. Pathol. 12: 73). Elicitor of soybean has a characteristic structure; it has β -1,6 linked glucan of various lengths as a principal chain from which β -1,3 linked glucan side chains are branched [J.K. Sharp et al. (1984) J. Biol. Chem. 259: 11321; M. Yoshikawa (1990) Plant Cell Technology 1.2: 695]. A receptor specific for a glucan elicitor derived from a soybean pathogenic mold fungus Phytophthora megasperma f. sp. glycinea is believed to be a protein which plays an important role in the synthesis and accumulation of the antibiotic agent glyceollin. A method for the purification of the elicitor receptor specific to that elicitor [E.G. Cosio et al., (1990) FEBS 264: 235; E.G. Cosio et al. (1992) Eur. J. Biochem. 204: 1115, T. Frey et al. (1993) Phytochemistry 32: 543] and a partial amino acid sequence for the elicitor receptor (Kakitani et al., Japanese Unexamined Patent Publication No. 6-321995) have been disclosed. However, no gene coding for the glucan elicitor receptor has been known. If a gene coding for elicitor receptor is found, it will be possible to create plants having resistance to pathogenic fungi by incorporating the gene into a chromosome of plants and expressing the elicitor receptor in the plants. Thus, improvement of the productivity of agricultural products can be expected.

It is an object of the present invention to provide a plant into which a gene coding for a glucan elicitor receptor has been transferred.

It is another object of the present invention to provide a method for creating a plant into which a gene coding for a glucan elicitor receptor has been transferred.

### DISCLOSURE OF THE INVENTION

As a result of intensive and extensive researches toward the resolution of the above assignment, the present inventors have succeeded in cloning a glucan elicitor receptor gene from a soybean cDNA library, transferring this gene into a tobacco plant and expressing it in the plant. Thus, the present invention has been achieved. The present invention provides a method for creating a plant having resistance to pathogenic fungi, comprising incorporating a DNA sequence coding for a glucan elicitor receptor into a chromosome of a plant and expressing the glucan elicitor receptor in the plant. The present invention also provides a plant having resistance to pathogenic fungi, characterized in that a DNA sequence coding for a glucan elicitor receptor has been transferred into the plant and the glucan elicitor receptor is expressed in it.

### BRIEF DESCRIPTION OF THE DRAWINGS

Figure 1 shows SDS-polyacrylamide gel electrophoresis patterns of three purification steps.

Figure 2 shows the maps of plasmids pER23-1 and pER23-2.

Figure 3 shows the procedure for constructing plasmid pKV1-ER23.

Figure 4 shows a transient increase in intracellular Ca²⁺ concentration after the addition of an elicitor to cultured soybean cells.

Figure 5 shows a transient increase in intracellular Ca²⁺ concentration after the addition of an elicitor to transformed cultured tobacco cells.

Figure 6 shows elicitor-binding activities of full- or partial length ER expressed in E. coli.

Figure 7 shows the inhibition of the binding of an elicitor with an elicitor binding protein in a soybean cotyledon membrane fraction by an antibody against an elicitor-binding domain.

Figure 8 shows the inhibition of an elicitor-induced phytoalexin accumulation in soybean cotyledons by an antibody against an elicitor-binding domain.

Figure 9 shows the resistance of transformed tobacco plants to P. nicotianae.

Figure 10 presents photographs showing the resistance of transformed tobacco plants to R. solani.

Figure 11 shows the resistance of transformed tobacco plants to R. solani.

Figure 12 shows the resistance of transformed tobacco plants to P. nicotianae in an inoculation test using zoospores from P. nicotianae.

Figure 13 shows the structure of plasmid pPG1.

Figure 14 shows the results of determination of the glucanase activity of a glutathione-S-transferase/kidney bean glucanase fusion protein.

### PREFERRED EMBODIMENTS OF CARRYING OUT THE INVENTION

Glucan elicitor receptor is a protein involved in the production of phytoalexins and which functions as a receptor for glucan elicitors derived from glucan, a cell wall component of fungi. Its function is to signal microsomes and nuclei to increase the phytoalexin content in cells; this function is effected through binding to a glucan elicitor generated by the cleavage of a part of mycelial walls of a pathogen by β-1,3-glucanase in plant cells when the pathogen, e.g. a microorganism of the genus Phytophthora has invaded into plant tissues. As a specific example of glucan elicitor receptor, one having an amino acid sequence as substantially shown in SEQ ID NO: 1 has been found by the present inventors (Japanese Patent Application No. 6-136100). The "amino acid sequence as substantially shown in SEQ ID NO: 1" includes amino acid sequences as shown in SEQ ID NO: 1 in which there may be the deletion, replacement or addition of an amino acid(s), provided that they maintain the function of a glucan elicitor receptor.

The glucan elicitor receptor can be produced, for example, by a partially modified Cosio's method (E.J.B. (1992) 204: 1115). Briefly, the roots, leaves and stems of soybean, preferably variety green homer are homogenized and a membrane fraction is collected from the resulting slurry, purified by ion-exchange chromatography and further purified by affinity chromatography using an elicitor as a ligand. The elicitor used in the affinity chromatography is preferably derived from Phytophthora megasperma f. sp. glycinea race 1 (ATCC34566) because it shows incompatibility for green homer (i.e., resistance to the pathogen).

The amino acid sequence of the glucan elicitor receptor thus prepared can be determined as follows:

The purified glucan elicitor receptor is transferred on a PVDF membrane (Millipore Co.) by electroblotting and digested with lysylendopeptidase (AP-I). The fragmented peptides are recovered from the PVDF membrane and fractionated by reversed-phase HPLC ( µ - Bondasphere 5 µ C8). The peak fractions are analyzed with a gas-phase protein sequencer (Applied Biosystems Co.).

The glucan elicitor receptor is useful in the elucidation of resistance mechanism of plants to fungi and the development of elicitor derivatives capable of inducing resistance to fungi, and it can be used as an antigen for the production of antibodies against glucan elicitor receptors.

DNA sequences coding for a glucan elicitor receptor have preferably at least one stop codon (e.g., TAG) adjacent to the 3' end.

More specifically, DNA molecules containing nucleotide sequences coding for a glucan elicitor receptor having an amino acid sequence as substantially shown in SEQ ID NO: 1 can be used. The "DNA molecules containing nucleotide sequences coding for a glucan elicitor receptor" include all degenerate isomers. The term "degenerate isomers" means DNA molecules coding for the same polypeptide with different degenerate codons. If a DNA molecule having the nucleotide sequence of SEQ ID NO:2 is taken as an example, a DNA molecule in which a codon for any amino acid, e.g., AAC for Asn is changed to a degenerate codon AAT is called a degenerate isomer. Examples of such degenerate isomers include DNA molecules containing the nucleotide sequence shown in SEQ ID NO: 2. DNA sequences containing nucleotide sequences coding for a glucan elicitor receptor, which are incorporated in plasmid pER23-1, may be used. E. coli DH5α EKB633 transformed with plasmid pER23-1 was deposited with the National Institute of Bioscience and Human-Technology, the Agency of Industrial Science and Technology, on June 15, 1994 under Accession Number FERM BP-4699 (1-3, Higashi 1 chome Tsukuba-shi Ibaraki-ken 305, JAPAN).

The DNA sequences coding for a glucan elicitor receptor may optionally bind to an ATG codon for initiation methionine together with a translation frame in the upstream portion toward the 5' end and also bind to other DNA molecules having appropriate lengths as non-translation regions in the upstream portion toward the 5' end and the downstream portion toward the 3' end.

The DNA sequences coding for a glucan elicitor receptor can be present typically in the form of parts of constituents of plasmid or phage DNA molecules or in the form of parts of constituents of plasmid, phage or genomic DNA molecules which are introduced into microorganisms (particularly, bacteria including E. coli and Agrobacterium), phage particles or plants.

In order to express stably the DNA sequences coding for a glucan elicitor receptor in plants, a promoter, a DNA molecule (ATG) encoding the initiation codon and a terminator may be added to the DNA sequences in appropriate combinations. Examples of the promoter include the promoter of genes encoding ribulose-1,5-biphosphate carboxylase small subunit (Fluhr et al., Proc. Natl. Acad. Sci. USA (1986) 83:2358), the promoter of a nopaline synthase gene (Langridge et al., Plant Cell Rep. (1985) 4:355), the promoter for the production of cauliflower mosaic virus 19S-RNA (Guilley et al., Cell (1982) 30:763), the promoter for the production of cauliflower mosaic virus 35S-RNA (Odell et al., Nature (1985) 313:810) and the like. Examples of the terminator include the terminator of a nopaline synthase gene (Depicker et al., J. Mol. Appl. Gen. (1982) 1:561), the terminator of an octopine synthase gene (Gielen et al., EMBO J. (1984) 3:835) and the like.

The DNA molecule coding for a glucan elicitor receptor can be obtained by a method comprising the steps of chemically synthesizing at least a part of the DNA molecule according to a conventional procedure of synthesis of nucleic acids and obtaining a desired DNA molecule from an appropriate cDNA library using the synthesized DNA molecule as a probe by a conventional method, for example, an immunological method or a hybridization method. Some plasmids, various kinds of restriction enzymes, T4 DNA ligase and other enzymes for use in the above method are commercially available. The DNA cloning, the construction of plasmids, the transfection of a host, the cultivation of the transfectant, the recovery of DNA molecules from the culture and other steps can be performed by the methods described in Molecular Cloning, J. Sambrook et al., CSH Laboratory (1989), Current Protocols in Molecular Biology, F. M. Ausubel et al., John Wiley & Sons (1987) and others.

More specifically, the DNA molecules coding for a glucan elicitor receptor can be obtained as follows:

Two kinds of partial amino acid sequences are selected from the amino acid sequences of a glucan elicitor receptor. Primers consisting of combinations of all bases which can encode the C terminus of the selected partial sequence and primers consisting of combinations of all bases which can encode the N terminus of the selected partial sequence are prepared. These synthesized primers are used as mixed primers to perform two PCR using DNA molecules of an appropriate soybean cDNA library as a template. Subsequently, two amplified fragments of given lengths whose amplification is expected (these fragments correspond to DNA molecules encoding the above two partial amino acid sequences) are picked up and the nucleotide sequences thereof are determined. On the basis of the determined nucleotide sequences, a primer having nucleotide sequences coding for the C terminus of an amino acid partial sequence positioned at the C terminal side of the glucan elicitor receptor and a primer having nucleotide sequences coding for the N terminus of an amino acid partial sequence positioned at the N terminal side of the glucan elicitor receptor are synthesized. These two synthesized primers are used to perform a PCR using the DNA molecules of the above soybean cDNA library as a template. The resulting amplified fragments are used as probes to hybridize the aforementioned soybean cDNA library, thereby yielding DNA molecules containing nucleotide sequences coding for the glucan elicitor receptor.

The obtained DNA molecules containing nucleotide sequences coding for the glucan elicitor receptor can be sequenced by any known methods, for example, the Maxam-Gilbert method (Methods Enzymol., 65:499, 1980), a dideoxynucleotide chain termination method using M13 phage (J. Messing, et al., Gene, 19:269, 1982) and the like.

Since the results of various studies on glucan elicitor suggest that a glucan elicitor receptor plays an important role in resistance to fungi in plants, it is expected that the DNA sequences coding for a glucan elicitor receptor can impart fungal resistance to plants if they are introduced and expressed in plant cells (particularly higher plant cells) which have no glucan elicitor receptor according to a conventional procedure. It has been proposed that fungi capable of infecting plants have generally suppressors, thereby acquiring an ability to suppress the fungal resistance of the plants. It is expected that new plants having resistance to fungi can be developed by introducing and expressing the DNA sequences coding for a glucan elicitor receptor such that the glucan elicitor receptor works or by modifying the DNA molecules or by regulating their expression levels.

Moreover, if the DNA sequences coding for a glucan elicitor receptor are introduced and expressed in plant cells, particularly in higher plant cells, together with fungal resistance enhancing genes or characters such as the gene of glucanase which imparts fungal resistance to plants, it is expected that higher fungal resistance can be imparted to plants than in the case where the gene of glucanase is introduced. Specific examples of a DNA sequence coding for a glucanase include a DNA comprising a nucleotide sequence coding for a glucanase having an amino acid sequence as substantially shown in SEQ ID NO: 3 or 34. The "DNA comprising a nucleotide sequence coding for a glucanase" is intended to include all degenerate isomers. As a specific example of such degenerate isomers, a DNA comprising the nucleotide sequence as shown in SEQ ID NO: 4 or 33 may be mentioned.

Vectors used for introducing the DNA sequences coding for a glucan elicitor receptor may be constructed such that the glucan elicitor receptor can be stably expressed in plants. More specifically, a promoter, a DNA molecule encoding the initiation codon (ATG) and a terminator may be added to the DNA sequences coding for a glucan elicitor receptor in appropriate combinations. Examples of the promoter include the promoter of genes encoding ribulose-1,5-biphosphate carboxylase small subunit (Fluhr et al., Proc. Natl. Acad. Sci. USA (1986) 83:2358), the promoter of a nopaline synthase gene (Langridge et al., Plant Cell Rep. (1985) 4:355), the promoter for the production of cauliflower mosaic virus 19S-RNA (Guilley et al., Cell (1982) 30:763), the promoter for the production of cauliflower mosaic virus 35S-RNA (Odell et al., Nature (1985) 313:810) and the like. Examples of the terminator include the terminator of a nopaline synthase gene (Depicker et al., J. Mol. Appl. Gen. (1982) 1:561) and the terminator of an octopine synthase gene (Gielen et al., EMBO J. (1984) 3:835).

The DNA molecules coding for a glucan elicitor receptor can be introduced into plant cells by any usual known methods, for example, the method described in "Plant genetic transformation and gene expression; a laboratory manual", J. Draper, et al. eds., Blackwell Scientific Publications, 1988. Examples of the methods include biological methods such as those using viruses or Agrobacteria and physicochemical methods such as electroporation, a polyethylene glycol method, microinjection, particle gun method, dextran method and the like.

When the plant to be transformed is a dicotyledonous plant, the method using Agrobacterium is generally preferable. When the plant to be transformed is a monocotyledonous plant or a dicotyledonous plant that is not susceptible to infection with Agrobacterium, a physical/chemical method such as electroporation is preferable. As a plant material into which a DNA of interest is to be transferred, an appropriate material may be selected from leaves, stems, roots, tubers, protoplasts, calluses, pollen, seed embryos, shoot primordia, etc. according to the method of transfer or the like.

When a DNA of interest is to be transferred into cultured plant cells, protoplasts are generally used as a material and the DNA is transferred thereinto by a physical/chemical method such as electroporation, the polyethylene glycol method or the like. On the other hand, when a DNA of interest is to be transferred into plant tissues, leaves, stems, roots, tubers, calluses, pollen, seed embryos, shoot primordia or the like are used as a material; preferably, leaves or stems are used. The DNA is transferred into such plant tissues by a biological method using a virus or Agrobacterium or a physical/chemica l method such as the particle gun method, microinjection or the like; preferably, a biological method using Agrobacterium is used.

In order to regenerate a plant from those plant tissues or plant cells into which a DNA sequence coding for a glucan elicitor receptor has been transferred, these transformed plants or cells may be cultured in a medium such as hormone-free MS medium, if they are derived from tobacco. The resultant seedlings which are rooting may be transferred to soil to give grown-up plants.

As plants which can be imparted resistance or enhanced resistance to pathogenic fungi by transferring a DNA sequence coding for a glucan elicitor receptor and expressing the glucan elicitor receptor by the methods described above, plants which are susceptible to infection with pathogenic fungi containing glucan in the cell walls may be mentioned. Specific examples of these plants include, but are not limited to, solanaceous plants and leguminous plants. More specifically, these plants include, but are not limited to, tobacco, soybean, potato, rice, chrysanthemum and carnation.

As pathogenic fungi, those containing glucan in the cell walls are embraced by the present invention. Specific examples of the pathogenic fungi include, but are not limited to, the genuses Phytophthora, Rhizoctonia, Pyricularia, Puccinia, Fusarium, Uromyces and Botrytis. More specifically, the pathogenic fungi include, but are not limited to, Phytophthora nicotianae, Rhizoctonia solani, Pyricularia oryzae, Puccinia horiana, Fusarium oxysporum, Uromyces dianthi and Botrytis cinerea.

According to the present invention, the DNA sequence coding for a glucan elicitor receptor transferred into a plant can be inherited to subsequent generations through seeds. Thus, the transferred DNA sequence is also present in those seeds which are formed from the pollen or ovaries of the plant of the invention, and the inherited character can be transmitted to the progeny. Accordingly, the plant of the invention into which a DNA sequence coding for a glucan elicitor receptor has been transferred can be propagated through seeds without losing its resistance to pathogenic fungi. The plant of the invention can also be propagated by a mass propagation method using plant tissue culture or by conventional techniques such as cutting, layering, grafting, division, etc. without losing its resistance to pathogenic fungi.

Whether a transformed plant has resistance to fungi or not can be examined by the following test methods.

Resistance to a Phytophthora fungus can be assayed by inoculating a fungal mycelium directly into plants and observing the expansion of lesions. Alternatively, the resistance may be assayed by inoculating zoospores from the fungus and observing the formation and expansion of lesions.

Resistance to a soil fungus can be assayed by mixing cultured fungal cells with soil, sowing seeds or setting plants on the soil, and observing a phenomenon of damping-off.

The present invention will now be explained in greater detail with reference to the following examples which are by no means intended to limit the scope of the present invention. In the following examples, a glucan elicitor receptor is abbreviated to "ER".

### Example 1

### Purification of soybean root-derived glucan elicitor receptor

### 1) Measurement of glucan elicitor binding activity of ER

A complex of an elicitor (average molecular weight: 10,000) and tyramine (TOKYO KASEI KOGYO CO., LTD.) was synthesized by the method of Jong-Joo Cheong (The Plant Cell (1991) 3: 127). The elicitor-tyramine complex was labelled with iodine using chloramine T.

A sample (protein amount < 500 µg) was suspended in 500 µl of an assay buffer (50 mM Tris-HCl pH7.4, 0.1 M saccharose, 5 mM MgCl₂, 1mM PMSF and 5 mM EDTA) and incubated at 0°C for 2 hours. The iodine-labelled elicitor-tyramine complex in an amount of 7.0 nM (70 Ci/mmol, the number of moles is calculated on the assumption that the molecular weight of the elicitor is 10,000, and this applies to the following description) was added to the suspension and the mixture was incubated at 4 °C for 2 hours. The reaction solution was filtered through Whatman GF/B as treated with a 0.3% aqueous solution of polyethylene imine for at least 1 hour. The residue was washed 3 times with 5 ml of an ice-cold buffer (10 mM Tris-HCl pH 7.0, 1 M NaCl, 10 mM MgCl₂). The radio activity retained on the filter was counted with a gamma counter (count A). In order to eliminate the effect of non-specific binding, the same procedure as above was performed except that 17 µM of the elicitor was added to the same sample, the mixture was suspended in the assay buffer and the suspension was incubated at 0 °C for 2 hours. The obtained count was subtracted from the count A to give a count (Δ cpm) of elicitor-specific binding. The resulting count (Δ cpm) was divided by the total number of counts and then multiplied by the total amount of elicitor used in the experiment to calculate the amount of the elicitor-binding protein (in moles).

The purity of ER was checked by the above method.

### 2) Purification of soybean root-derived ER

Soybean (Glycine max cv. Green Homer) seeds (Takayama Seed Co.) were cultured on vermiculite for 1 week and then aquicultured for 15 days to harvest roots (about 40 kg, wet weight). The harvested roots were stored at -80 °C until they were used for the purification of ER. An ice-cold buffer (25 mM Tris-HCl pH 7.0, 30 mM MgCl₂ , 2 mM dithiothreitol, 2.5 mM potassium metabisulfite and 1 mM PMSF) was added to the roots (2 kg, wet weight) in an amount of 1.25 L and the mixture was homogenized with a Waring Blender for 2 minutes.

The resulting slurry was filtered through a Miracloth (Calbiochem Co.) and the filtrate was centrifuged at 9,000 rpm at 4 °C for 15 minutes. The supernatant was ultracentrifuged at 37,000 rpm at 4°C for 20 minutes. The precipitate was suspended in 160 ml of an ice-cold buffer (25 mM Tris-HCl pH 7.4, 0.1 M sucrose, 5 mM MgCl₂, 1 mM PMSF and 5 mM EDTA) to give a membrane fraction. An ampholytic detergent ZW3-12 (Boehringer Co.) was added to the membrane fraction to give a final concentration of 0.25% for solubilization of ER from the membrane fraction and the mixture was stirred at 8 °C for 30 minutes. The resulting mixture was ultracentrifuged at 37,000 rpm at 4 °C for 20 minutes to collect the supernatant containing the solubilized ER (soluble fraction). The soluble fraction (165 ml) was dialyzed against 2 l of a buffer (50 mM Tris-HCl pH 8.0, 0.2% ZW3-12, 4 °C ) 4 times. Five milliliters of Protrap (TAKARA SHUZO CO., LTD.) was added to the sample and the mixture was stirred at 8 °C for 30 minutes to remove proteases from the sample and to stabilize ER. The resulting mixture was centrifuged at 2,800 rpm at 4 °C for 2 minutes to collect the supernatant. The obtained supernatant (160 ml) was concentrated to about 50 ml using an ultrafiltration membrane YM-10 (Amicon Co.) and the concentrate was dialyzed against an A buffer (50 mM Tris-HCl pH 8.0, 0.1 M sucrose, 5 mM MgCl₂, 1 mM PMSF, 5 mM EDTA and 0.2% ZW3-12, 4 °C).

The dialysate was applied to Q-Sepharose HP 26/10 (Pharmacia Co.) and ER was eluted in a linear gradient of 0-1 M NaCl (Q-Sepharose active fraction). The ER was eluted at a NaCl concentration of about 0.45 M. The Q-Sepharose active fraction was diluted 3 folds with A buffer and the diluted fraction was applied to Mono Q 10/10 (Pharmacia Co.). The ER was eluted in a linear gradient of 0-1 M NaCl (Mono Q active fraction, 8 ml). The ER was eluted at a NaCl concentration of about 0.25 M.

The ER was purified with an affinity gel using an elicitor as a ligand as follows:

Elicitor was prepared according to N. T. Keen with some modifications (Plant Physiol. (1983) 71: 460, Plant Physiol. (1983) 71: 466). Briefly, the mycelial wall of pathogenic Phytophthora megasperma f. sp. glycinea race 1 (ATCC34566) was treated with zymolyase 100T (KIRIN BREWERY CO., LTD.) to liberate an elicitor. After the treatment, Zymolyase 100T was eliminated by the adsorption on CM-cellulose packed in a column. The resulting passage-through fraction was purified with a gel permeation chromatography G-75 (Pharmacia Co.) to collect an elicitor fraction whose average molecular weight was 10,000 Da. The glyceollin-inducing elicitor activity of the collected fraction was determined by the method of M. Yoshikawa (Nature (1978) 257:546). The addition of 8 µg of the elicitor to soybean cotyledons resulted in the induction of about 550 µg of glyceollin after 24 hours incubation.

In order to eliminate non-specific adsorption on the gel carrier, Mono Q active fraction was collected and stirred with about 33 mg of maltose-coupled glass gel (bed volume: about 100 µl) at 8 °C for 1 hour. The gel was precipitated by centrifugation (1,000 rpm, 4 °C , 2 minutes) to collect the supernatant (maltose-coupled glass gel passage-through fraction). The maltose-coupled glass gel was prepared by the method of A.M. Jeffrey et al. (Biochem. Biophys. Res. Commun. (1975) 62: 608). Briefly, 120 mg of maltose and 6 g of Glass Aminopropyl (Sigma Co.) were suspended in 36 ml of H₂O and the suspension was stirred at room temperature overnight. To the resulting suspension was added 36 ml of ethanol. Immediately thereafter a solution of sodium borohydride (864 mg) in ethanol (72 ml) was added to the mixture. The resulting mixture was sonicated for 2 minutes and stirred at room temperature for 5 hours. Water (288 ml) was added to the reaction mixture and the resulting mixture was cooled with ice and adjusted to pH 5.6 with acetic acid. The gel was washed with about 1.8 L of H₂O to remove the free maltose. Maltose contained in the washing solution was determined quantitatively by the method of J. H. Roe (J. Biol. Chem. (1955) 212:335) using an anthrone reagent. An amount of the gel-coupled maltose was estimated from the amount of the maltose contained in the washing solution. As a result, it was found that 60 mg of maltose was coupled to 6 g of Glass Aminopropyl.

About 17 mg of the elicitor-coupled glass gel (bed volume: about 50 µl) was added to 8 ml of the maltose-coupled glass gel passage-through fraction and the mixture was stirred gently at 8 °C overnight. The gel was collected by centrifugation (1,000 rpm, 4 °C , 2 minutes) and washed with 2 bed volumes of A buffer 2 times. The gel was washed additionally with 4 bed volumes of 0.1% SDS 3 times to collect gel-coupled ER (elicitor-coupled glass gel eluted fraction). The elicitor-coupled glass gel was prepared by the method of A.M. Jeffrey et al. (Biochem. Biophys. Res. Commun. (1975) 62: 608). Briefly, elicitor (37 mg) and Glass Aminopropyl (490 mg) were suspended in 6 ml of H₂O and stirred at room temperature overnight. Ethanol (6 ml) was added to the suspension and a solution of sodium borohydride (144 mg) in ethanol (12 ml) was added immediately thereafter. The mixture was sonicated for 2 minutes and stirred at room temperature for 5 hours. To the resulting mixture was added 48 ml of H₂O. The mixture was cooled with ice and adjusted to pH 5.6 with acetic acid. The free elicitor was determined quantitatively with an anthrone reagent. The amount of the gel-coupled elicitor was estimated from the amount of the free elicitor contained in the washing solution. As a result, it was found that 34 mg of the elicitor was coupled to 490 mg of Glass Aminopropyl.

The protein and ER amounts in the above steps for purification are summarized in Table 1.

**Table 1**

| Protein and ER Amounts in the Steps for Purification (Soybean roots weighing 40 kg on a wet basis were used as a starting material) | | |
|---|---|---|
| | Protein (mg) | ER (pmol) |
| Membrane Fraction | 17900 | 30 |
| Soluble Fraction | 2000 | 214 |
| Q-Sepharose Active Fraction | 190 | 205 |
| Mono Q Active Fraction | 49 | 233 |
| Maltose-Coupled Glass Gel Passage-through Fraction | 45 | 220 |
| Elicitor-Coupled Glass Gel Eluted Fraction | 0.004* | 45 |

| | | |
|---|---|---|
| * : Estimated from the band intensity obtained by silver stain after SDS-PAGE. | | |

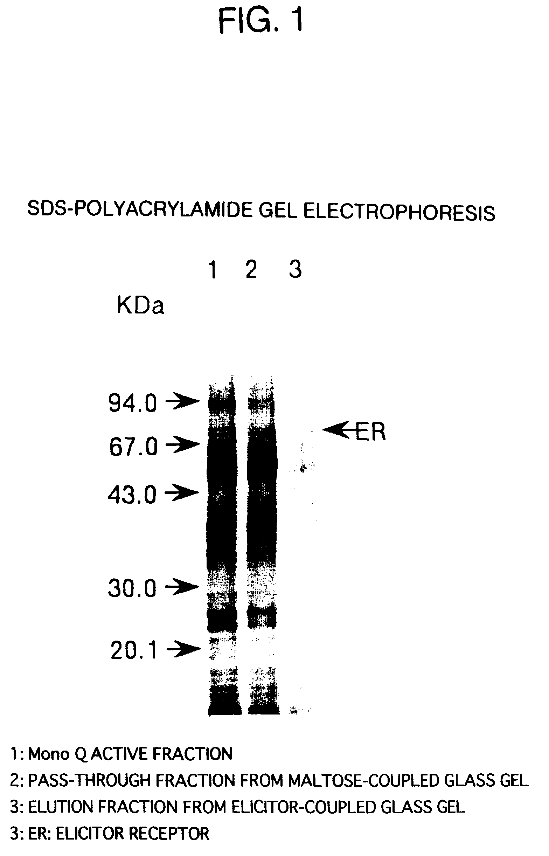

The Mono Q active fraction, passage-through fraction from maltose-coupled glass gel and eluted fraction from elicitor-coupled glass gel (10 µl each) were electrophoresed on an electrophoretic gradient gel, SDS-PAGE plate 10/20 (Daiich Kagaku Yakuhin Co.) and stained with silver (Daiich Kagaku Yakuhin Co.) The electrophoresis patterns are shown in Figure 1. In Figure 1, lane 1 is the Mono Q active fraction, lane 2; the passage-through fraction from the maltose-coupled glass gel and lane 3; the eluted fraction from the elicitor-coupled glass gel. Figure 1 shows that the ER bands were detected at a molecular weight of about 70,000 Da.

The protein of about 70,000 in molecular weight was labelled with iodine-125 by using an ¹²⁵I-labelled complex of a photoaffinity reagent SASD (Pierce Co.) and the elicitor. The SDS-PAGE band of the membrane fraction was transferred on a PVDF membrane by western blotting and incubated with the same ¹²⁵I-labelled elicitor as used in measuring the elicitor-binding activity of ER on the PVDF membrane so that the protein of about 70,000 Da in molecular weight was labeled with iodine-125. These facts reveal that the protein of about 70,000 Da in molecular weight had an elicitor-binding activity.

About 4 µg of ER was purified from about 40 kg by wet weight of the soybean root by the above method.

### 3) Analysis of ER-fragmented peptides

The ER was fragmented by protease digestion to peptides. The amino acid sequences of the fragmented peptides were determined by the method of Iwamatsu (Akihiro Iwamatsu, Seikagaku (1991) 63: 139, A. Iwamatsu, Electrophoresis (1992) 13: 142). A solution of the ER purified by the above method was concentrated to about 100 µl with Centricon-30 (Amicon Co. ) and subjected to a 10-20% polyacrylamide SDS electrophoresis. The resulting protein bands were transferred on a PVDF membrane (Millipore Co.) with an electroblotting apparatus (Sartrius Co.). The bands transferred on the PVDF membrane were stained with 0.1% Ponceau S (Sigma Co. )/1% acetic acid. The main band of 70,000 Da in molecular weight was sectioned and decolored with 0.5 mM NaOH. This band was reductively S-carboxymethlated. Lysylendopeptidase (AP-1) was added to the resulting band at an enzyme:substrate (mol:mol) ratio of 1:100 and the mixture was reacted at 30 °C for 16 hours. The resulting fragmented peptides were applied to a µ-Bondasphere 5µ C8-300 Å (2.1 x 150 mm, Waters) column equilibrated with 98% solvent A and 2% solvent B and eluted in a 2-50% linear gradient of solvent B for 30 minutes at a flow rate of 0.25 ml/minute (solvent A: 0.05% TFA solution, solvent B: 0.02% TFA in 2-propanol:acetonitrile=7:3 (v/v)). Eluted peptides were detected by absorbance at 214 nm and each peak fraction was collected manually. The obtained peak fractions were analyzed with a gas-phase protein sequencer (Model 470A of Applied Biosystems). As a result of analysis of all the peak fractions obtained, the following amino acid sequences of the fragmented peptides were clearly determined. #1:Val Asn Ile Gln Thr Asn Thr Ser Asn Ile Ser Pro Gln (N-terminus) (SEQ ID NO: 5) #5:Lys Ser Ile Asp Gly Asp Leu Val Gly Val Val Gly Asp Ser (SEQ ID NO: 6) #6:Lys Tyr Lys Pro Gln Ala Tyr Ser Ile Val Gln Asp Phe Leu Asn Leu Asp (SEQ ID NO:7) #7:Lys Thr Asp Pro Leu Phe Val Thr Trp His Ser Ile Lys (mix sequence) (SEQ ID NO: 8)

### Example 2

### Cloning of soybean ER gene

### 1) Preparation of soybean mRNA

Soybean (Glycine max cv. Green Homer) seeds (Takayama Seed Co.) were cultured on vermiculite for 1 week and aquicultured for 15 days to harvest roots (about 40 kg, wet weight). A portion of the harvested roots was stored at -80°C until it was used. Total RNA was obtained by the method of Ishida (Cell Technology Laboratory Manipulation Manual, Kodansha Scientific). Briefly, the stored roots (28.5 g, wet weight) were ground on a mortar while adding liquid nitrogen. To the obtained powder, 35.6 ml of a GTC solution held at 65°C was added and the mixture was homogenized with a Waring Blender. The resulting suspension was centrifuged at 6,000 rpm at room temperature for 15 minutes to collect 40 ml of the supernatant. The supernatant was layered gently on a cushion solution of cesium in a centrifuge tube and centrifuged at 35,000 rpm at 25 °C for 20 hours. The resulting precipitate was dissolved in 9 ml of TE/0.2% SDS. After phenol/chloroform extraction was conducted 2 times, total RNA (4.37 mg) was recovered by ethanol precipitation.

The obtained total RNA (2.2 mg) was used for purification with oligotex dT30 (Japan Roche Co.) according to the manual and then 68 µg of purified poly(A)+RNA was obtained.

### 2) Preparation of soybean cDNA library

cDNA molecules were synthesized from 5 µg of the poly(A)+RNA with a cDNA synthesis kit (Pharmacia Co.) according to the manual. The synthesized cDNA fragments were ligated to lambda phage vector λ gt10 (Stratagene Co.) with T4 ligase (TAKARA SHUZO CO., LTD.). Gigapack (Stratagene Co.) was used to package a DNA mixture into the phage particles to prepare a soybean cDNA library of about 1.5 x 10⁶ pfu. The library was amplified to 160 ml of a soybean cDNA library of 1.6 x 10¹¹ pfu/ml.

Total DNA in the cDNA library was prepared as follows:

Chloroform/isoamylalcohol (24:1) was added to 500 µl of a phage solution (1.6 x 10¹¹ pfu/ml) in an equal amount. The mixture was shaken for 30 seconds and centrifuged to collect the aqueous layer. The aqueous layer was extracted again with chloroform/isoamylalcohol (24:1). To the resulting aqueous layer were added 5 µl of a 3M sodium acetate solution (pH 5.4) and 125 µl of ethanol and the mixture was centrifuged to collect the precipitate. The precipitate was washed with a 70% ethanol solution and dissolved in a 10 mM Tris-HCl solution (pH 8) containing 1 µg/ml RNase A (Sigma Co.). This solution was used as a PCR template.

### 3) Amplification and Cloning of Soybean ER cDNA Fragments by PCR

The following four oligodeoxynucleotides (mixed primers U5, U7, U10 and U12) were synthesized with an automatic nucleic acid synthesizer (Model 394 of Applied Biosystems Co.) on the basis of the amino acid sequences of the fragmented peptides obtained in Example 1 (#5 and #6):
Primer U5 5'-AARAGYATHGAYGGNGA-3' (SEQ ID NO: 9)
Primer U7 5'-WRTCNCCNACNAC-3' (SEQ ID NO: 10)
Primer U10 5'-GTNAAYAARATNCARAC-3' (SEQ ID NO: 11)
Primer U12 5'-ARRTTNAGRAARTCYTC-3' (SEQ ID NO:12)
   (R:A/G, Y:C/T, W:A/T, H:A/C/T, N:A/G/T/C)

The total DNA in 0.5 µg of the cDNA library was dissolved in 79 µl of distilled water. Either a combination of primers U5 and U7 or a combination of primers U10 and U12 (100 pmol each) and 0.5µl of Taq DNA polymerase (TAHARA SHUZO CO., LTD.) were added to 8 µl of 2.5 mM dNTP in 10 µl of a 10 x PCR buffer (attached to Taq DNA polymerase of TAKARA SHUZO CO., LTD.) to give a final amount of 100 µl. PCR reaction was performed with a Gene Amp PCR System 9600 (Perkin-Elmer Co.) by 50 cycles of 1) denaturation at 94 °C x 30 seconds, 2) renaturation at 47°C x 30 seconds and 3) elongation at 72 °C x 1 minute. After the reaction, 15 µl of the reaction solution was electrophoresed on a 15% polyacrylamide gel. The gel was stained with a 0.5 µg/ml ethidium bromide solution for 10 minutes. The bands showing specifically amplified fragments of 40 bp and 47 bp whose amplification was expected were sectioned while observing under UV light. The gel sections were ground with a plastic bar and eluted with an elution buffer (0.5 M ammonium acetate, 10 mM magnesium acetate, 1 mM EDTA and 0.1% SDS) overnight to collect a DNA-containing solution.

The collected DNA fragments were cloned into plasmid pT7Blue(R) with a pT7Blue T-Vector Kit (Novagene Co.). The obtained plasmids p#5-1, 2, and p#6-1, 2, 3, 4, 5, 6, 7, 8 and 9 were sequenced with a fluorescence automatic DNA sequencer (Model 373A of Applied Biosystems Co.). The results showed that the resulting amplified DNA fragments other than the primers also encoded the amino acid sequences of fragmented peptides #5 and #6.

The following two oligodeoxynucleotides (mixed primers U18 and U19) were synthesized with an automatic nucleic acid synthesizer on the basis of the results of the DNA sequencing.
Primer U18 5'-AAGTAYAAGCCRCAAGCCTATTCA-3' (SEQ ID NO:13)
Primer U19 5'-ATCGCCRACAACMCCAA-3' (SEQ ID NO:14)
   (Y and R are as defined above, M:A/C)

The total DNA in 0.5 µg of the cDNA library was dissolved in 79 µl of distilled water. A combination of primers U18 and U19 (100 pmol each) and 0.5 µl of Taq DNA polymerase were added to 8 µl 2.5 mM dNTP in 10 µl of a 10 x PCR buffer to give a final amount of 100 µl. PCR reaction was performed by 40 cycles of 1) denaturation at 94°C x 30 seconds, 2) renaturation at 52°C x 30 seconds and 3) elongation at 72 °C x 1 minute. Fifteen microliters of the reaction solution was electrophoresed on a 1% agarose gel.

The gel was stained with a 0.5 µg/ml ethidium bromide solution for 15 minutes. The band showing a specifically amplified fragment of about 540 bp was sectioned while observing under UV light. The gel section was treated with Gene Clean II (Bio101 Co.) to collect a DNA-containing solution.

The collected DNA fragment was cloned into plasmid pT7Blue(R) with a pT7Blue T-Vector Kit. The obtained plasmid p#5-#6 was sequenced with a fluorescence sequencer. The results showed that the amplified DNA fragment consisted of 539 bp and encoded not only the amino acid sequences of fragmented peptides #5 and #6 at the both sides, but also peptide #7 in the amplified portion.

### 4) Screening and Cloning of Library by Hybridization

Plasmid #5-#6 into which the ER cDNA fragment was cloned was digested with restriction enzymes BamHI and PstI. A DNA fragment of about 540 bp was recovered and used as a probe. The recovered DNA fragment was labelled with [α -³²P] dCTP using a Megaprime DNA labelling system (Amersham Co.) according to the manual and the reaction solution was used in a hybridization experiment.

A phage of the cDNA library was infected with E. coli C600 hfl (Invitrogen Co.) and inoculated in a 10 mg/ml MgCl₂-containing L medium on plates of about 15 cm in diameter to form a total of about 1 x 10⁶ of plaques. The plaques were blotted on a nylon membrane (Hybond-N; Amersham Co.). The membrane was reacted with the ³²P-dCTP labeled ER cDNA fragment and positive phages detected by autoradiography were screened again in the same way to give about 30 phage clones having different signal intensities. Clone λ ER23 having the longest inserted DNA fragment was selected.

The λ phage DNA molecule was purified with a LambdaSorb (Promega Co.) from a solution of the positive clone λ ER23 isolated in the hybridization experiment. Ten microliters of a 10 x EcoRI cleavage buffer (restriction enzyme EcoRI 10 U) was added to 5 µg of the DNA solution to give a total amount of 100 µl and the mixture was reacted at 37 °C overnight. The reaction solution was electrophoresed on a 1% agarose gel. A band of about 2.3 kb was sectioned and treated with a Gene Clean II (Bio101 Co.) to collect a DNA-containing solution. Vector pBluescriptII KS- (0.02 µg) (Stratagene Co.) was cleaved with restriction enzyme EcoRI.

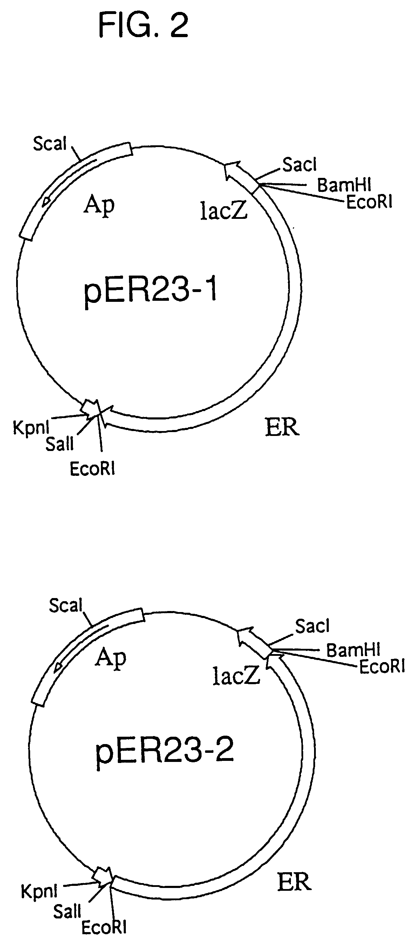

After the two DNA solutions were mixed, 2 µl of a 10 x ligase buffer and 0.2 µl of T4 DNA ligase (TAKARA SHUZO CO., LTD) were added to give a total amount of 20 µl. The mixture was reacted at 16 °C for 4 hours and the reaction mixture solution was used to transform E. coli DH5α (Gibco BRL Co.). A 2% agar plate medium was prepared with 25 ml of an L medium containing 50 µg/ ml ampicillin, 40 µg/ ml IPTG and 40 µg/ ml X-gal. The transformed E. coli was inoculated on the agar plate medium and grown at 37°C overnight. White colonies were selected from the formed colonies and cultured in 3 ml of an L medium containing 50 µg/ ml ampicillin at 37 °C for 8 hours. Plasmids were recovered from these bacterial cells by an alkaline method and determined whether they were clones into which a desired fragment was cloned with the restriction enzyme, thereby giving plasmids pER23-1 and pER23-2 (5225 bp) which had opposite orientations to the vector. The maps of plasmids pER23-1 and pER23-2 are shown in Figure 2.

### 5) Determination of the Nucleotide Sequence of the ER-encoding Clone

The DNA nucleotide sequences of plasmids pER23-1 and pER23-2 were determined in both orientations with a fluorescence sequencer by 1) using plasmids pER23-1 and pER23-2 digested by appropriate restriction enzymes, 2) using appropriate primers synthesized on the basis of the information about already determined nucleotide sequences, or 3) cleaving pER23-1 with restriction enzymes KpnI and XhoI and pER23-2 with restriction enzymes KpnI and ClaI and then using a kilosequence deletion kit (TAKARA SHUZO CO., LTD) to prepare plasmids having a deletion at intervals of about 200-300 bp. The DNA nucleotide sequence is shown in SEQ ID NO: 2 of the SEQUENCE LISTING. The results showed that the DNA fragment contained a 667 amino acid-encoding open reading frame of 2001 bp beginning at a nucleotide sequence corresponding to the N-terminal sequence ( fragmented peptide #1) sequenced with the amino acid sequencer. The amino acid sequence is shown in SEQ ID NO: 1 of the SEQUENCE LISTING. The amino acid sequence deduced from the resulting DNA nucleotide sequence was consistent with the previously determined amino acid sequence of the soybean ER.

In addition, highly homologous amino acid sequences were searched for with a nucleic acid and amino acid sequence analysis software package (MacVector: Kodak Co.) using a nucleic acid and amino acid sequence data base (Entrez: NCBI). However, no amino acid sequences were found to be highly homologous to the heretofore known sequences. Hence, it is clear that the prepared ER is a novel protein.

### Example 3

### Expression of the Soybean ER in Tobacco Plants

### 1) Construction of Plant Expression Plasmid pKV1-ER23

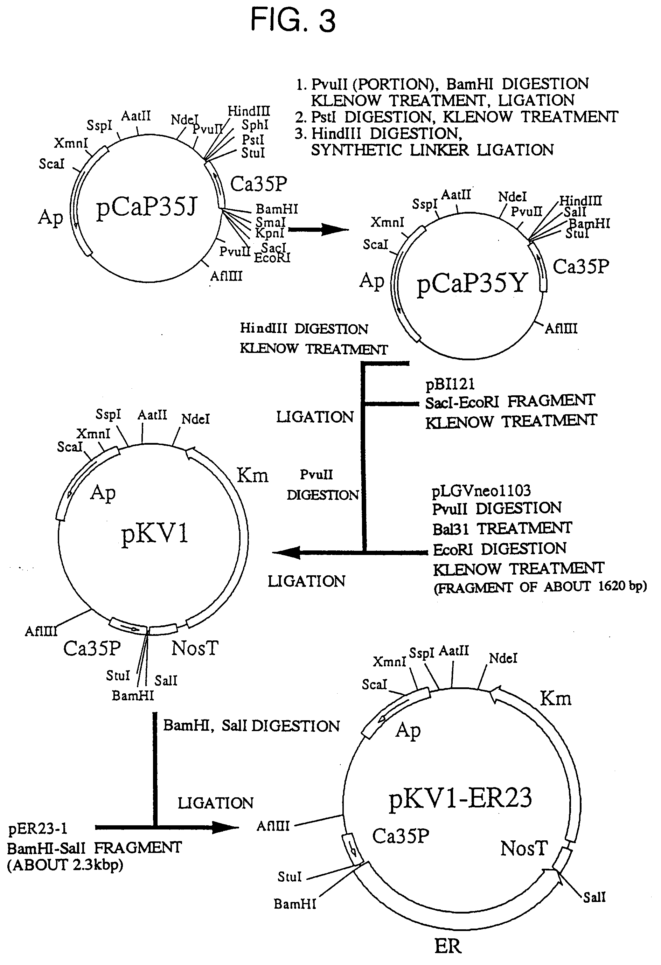

As shown in Figure 3, a plant expression vector pKV1 to be used in this example was prepared from cauliflower mosaic virus 355 promoter-containing plasmid pCaP35J (J. Yamaya et al. (1988) Mol. Gen. Genet. 211:520) as follows:

Plasmid pCaP35J was digested completely with restriction enzyme BamHI to delete a multi-cloning site present upstream of the 35S promoter. Following partial digestion with PvuII, a treatment was conducted with Klenow fragments (TAKARA SHUZO CO., LTD) to make blunt ends. The resulting plasmid DNA was circularized by ligation and introduced into E. coli DH5α . A desired plasmid was selected from the resulting clones. The selected plasmid was digested with restriction enzyme PstI to insert a multi-cloning site present downstream of the 35S promoter. A treatment was conducted with Klenow fragments to make blunt ends. The resulting plasmid DNA was digested with HindIII. The following synthetic linker DNAs were synthesized with an automatic nucleic acid synthesizer, annealed and ligated to the HindIII-digested plasmid. The resulting plasmid DNA was introduced into E. coli DH5α. Desired plasmid pCaP35Y (2837 bp) was selected from the obtained clones.

5'-GGAATTCGAGCTCGGTACCCGGGGATCCTCTAGAGTCGACCTGCAGGCATGCA-3'(SEQ ID NO:
15)
5'-CCTTAAGCTCGAGCCATGGGCCCCTAGGAGATCTCAGCTGGACGTCCGTACGTTCGA-3'(SEQ ID
NO:16)

In order to introduce a terminator of nopaline synthase into the pCaP35Y, plasmid pBI121 (Clontech Co.) was digested with SacI and EcoRI and the SacI-EcoRI fragment was treated with Klenow fragments to make blunt ends; then, the resulting fragment of pBI121 was ligated to plasmid pCaP35Y in which blunt ends were made at a HindIII site downstream of the 35S promoter. The resulting plasmid DNA was introduced into E. coli DH5α . A desired plasmid was selected from the obtained clones. In order to introduce a kanamycin-resistance cassette into the selected plasmid, the latter was digested with PvuII and ligated to a fragment (about 1620 bp) of pLGVneo1103 (R. Hain et al. (1985) Mol. Gen. Genet. 199: 161) that was obtained by the steps of cleavage at a PvuII site present downstream of the octopine synthase terminator, treatment with Bal31 (TAKARA SHUZO CO., LTD) to make a deletion, cleavage at a EcoRI site upstream of a nopaline synthase promoter, and the creation of a blunt end at both ends. The resulting plasmid DNA was introduced into E. coli DH5α . Desired plasmid, or plant expression vector pKV1 (4828 bp), was selected from the obtained clones.

The prepared pKV1 was digested at unique sites by restriction enzymes BamHI and SalI and ligated to the ER gene-containing fragment (i.e., the BamHI-SalI fragment of pEB23-1, about 2.3 kbp). The resulting plasmid DNA was introduced into E. coli DH5α . Desired ER-expression plasmid pKV1-ER23 (about 7.1 kbp) was selected from the obtained clones.

### 2) Transient Expression of ER in Cultured Tobacco Cells

The ER gene was introduced into cultured tobacco cells by electroporation for transient ER expression by a partial modification of Watanabe's method (Y. Watanabe (1987) FEBS 219: 65). The DNA molecules of the plasmid pKV1-ER23 were purified by an alkaline method. Cultured tobacco cells were obtained by the method of Hirai et al. (Plant Cell Cultivation Manual, Gakkai Shuppan Center, 1982) for use in the transient expression of ER. Tobacco seeds (variety Bright Yellow, provided by Professor Hirofumi Uchimiya of University of Tokyo) were sterilized with a 1% sodium hypochlorite solution and then germinated. The tobacco juvenile tissues just after the germination were transplanted in a tobacco cultivation agar medium (Murashige-Skoog medium (Flow Laboratories Co. ) supplemented with 2 ppm 2,4-dichlorophenoxyacetic acid, 3% sucrose and 8% agar) to induce calluses after 3 weeks. About 1 g of callus masses were suspended in 50 ml of a tobacco cultivation medium (Murashige-Skoog medium (Flow Laboratories Co.) supplemented with 2 ppm 2,4-dichlorophenoxyacetic acid and 3% sucrose) to prepare cultured cells. These tobacco cells were cultured until they entered a logarithmic growth phase. The cultured cells were collected by centrifugation (600 rpm, 3 minutes) and suspended in a solution consisting of 1% cellulase Onozuka (Yakult Co.), 1% Dricelase (Kyowa Hakko Co., Ltd.), 0.1% Pectriase (Seishin Seiyaku Co.) and 0.4 M D-mannitol (Wako Pure Chemicals Co., Ltd.) and which was adjusted to pH 5.7 with HCl. Reaction was performed at 30 °C for 90 minutes to prepare protoplasts. The reaction solution was washed with 0.4 M D-mannitol at 4 °C by 3 cycles of centrifugation to remove the enzyme solution. The operation of electroporation consisted of suspending 1 x 10⁶ cells in 0.8 ml of an electroporation solution (70 mM KCl, 5 mM MES and 0.3 M mannitol), mixing the suspension with 10 µg of the DNA molecules of pKV1-ER23 and treating the mixture with a genepluser (Biorad Co.) at 125 µF and 300 V in an electroporation cuvette (Biorad Co., electrode spacing: 0.4 cm). After the treatment, the solution was collected with a pasteur pipet and left to stand on ice for 30 minutes. Reaction was performed at 30 °C for 5 minutes and the reaction solution was resuspended in a protoplast medium (Murashige-Skoog medium (Flow Laboratories Co.) supplemented with 0.2 ppm 2,4-dichlorophenoxyacetic acid, 1% sucrose and 0.4 M mannitol and adjusted to pH 5.7). The cells were left to stand in the dark at 25°C overnight and collected by centrifugation (8,000 rpm, 3 minutes). Sixty microliters of a suspension buffer (25 mM Tris-HCl pH7.0, 30 mM MgCl₂ , 2 mM dithiothreitol, 2.5 mM potassium metabisulfite and 1 mM PMSF) were added to the cells and the mixture was stirred on a vortex for 3 minutes. The resulting sample was stored at -80°C until an elicitor-binding experiment was conducted.

For control, the above procedure was repeated except that the DNA molecule of pKV1 instead of pKV1-ER23 was introduced into tobacco cells.

### 3) Stable Expression of ER in Tobacco Suspension Cultured Cells

Transformed cultured tobacco cells capable of constant ER gene retention were selected as follows from the cultured tobacco cells capable of transient ER expression:

The protoplasts obtained in the preparation of the cultured tobacco cells capable of transient ER expression were suspended in a 1% agarose-containing protoplast medium (Murashige-Skoog medium (Flow Laboratories Co.) supplemented with 0.2 ppm 2,4-dichlorophenoxyacetic acid, 1% sucrose and 0.4 M mannitol and adjusted to pH 5.7). The suspension was dropped on a plate with a dropping pipet before the agarose was solidified, whereby the protoplasts were fixed in the bead-like solid medium. After the agarose was solidified, an agarose-free protoplast medium was added to the plate, thereby immersing the protoplast-fixing agarose medium in the liquid medium. After the protoplasts were cultured in the dark for 1 week, kanamycin was added to a final concentration of 100 µg/ml and the cultivation was continued. Transformants selected from the grown colonies were transferred in a kanamycin-containing liquid medium and cultured.

Two clones (I 1 and I 6) of cultured tobacco cells stably transformed by pKV1-ER23 and two clones (C 2-1 and C 2-4) of cultured tobacco cells stably transformed by pKV1 were obtained.

### 4) Elicitor-binding Activity Experiment

The elicitor-binding activity was measured as follows:

A complex of an elicitor and tyramine (TOKYO KASEI KOGYO CO., LTD.) was synthesized by the method of Jong-Joo Cheong (The Plant Cell (1991) 3: 127). The elicitor-tyramine complex was labelled with iodine-125 using chloramine T. The resulting sample (protein amount < 500 µg) was suspended in 500 µl of an assay buffer (50 mM Tris-HCl pH7.4, 0.1 M saccharose, 5 mM MgCl₂, 1mM PMSF and 5 mM EDTA) and incubated at 0°C for 2 hours. The iodine-labelled elicitor-tyramine complex in an amount of 100 nM (70 Ci/mmol) was added to the suspension and the mixture was incubated at 4 °C for 2 hours. The reaction solution was filtered through Whatman GF/B (as treated with a 0.3% aqueous solution of polyethylenimine for at least 1 hour) and washed 3 times with 5 ml of an ice-cold buffer (10 mM Tris-HCl pH 7.0, 1 M NaCl, 10 mM MgCl₂). The radio activity retained on the filter membrane was counted with a gamma counter (count A). In order to eliminate the effect of non-specific binding, the same procedure as above was performed except that 17 µM of the elicitor was added to the same sample, the mixture was suspended in the assay buffer and the suspension was incubated at 0 °C for 2 hours. The obtained count was subtracted from the count A to give a count (Δ cpm) of elicitor-specific binding. The resulting count (Δ cpm) was divided by the total number of counts and then multiplied by the total amount of elicitor used in the experiment to calculate the amount of the elicitor-binding protein (in moles).

As a result, a specific binding to the elicitor was observed in the tobacco cells transformed with the DNA molecule of pKV1-ER23, whereas no specific binding to the elicitor was observed in the control tobacco cells in which the DNA molecule of pKV1 was introduced (Table 2). This fact reveals that the gene obtained above encodes a protein having the elicitor-binding activity.

**Table 2**

| Elicitor-binding Activity of Cultured Tobacco Cells | | |
|---|---|---|
| Fraction | Transfoming DNA | Binding Activity (fmol/mg) |
| Transient Expression | pKV1 | < 0 |
| | pKV1-ER23 | 90.5 |

| Stable Expression | | |
|---|---|---|
| C2-1 | pKV1 | < 0 |
| C2-4 | pKV1 | < 0 |
| I 1 | pKV1-ER23 | 150 |
| I 6 | pKV1-ER23 | 196 |

### 5) Transient Increase in Intracellular Ca²⁺ Concentration in Transformed Tobacco Cultured Cells by Addition of Glucan Elicitor

Plants recognize the elicitor by a specific receptor thereto and then promote the accumulation of phytoalexin or induce hypersensitive reaction to prevent fungus invasion. It has been reported for some plants that the inflow of calcium ion into cells in the early phase of such resistance reactions is important (U. Conrath et al. (1991) FEBS LETTERS 279: 141, M. N. Zook et al. (1987 ) Plant Physiol. 84: 520, F. Kurosaki et al. (1987 ) Phytochemistry 26: 1919; C. L. Preisig and R. A. Moreau (1994) Phytochemistry 36: 857). A report has also been made suggesting that the inflow of calcium ion into cells triggers the promotion of the phytoalexin accumulation in soybean which the present inventors used to obtain ER (M. R. Stab and J. Ebel (1987) Archi. Biochem. Biophys. 257: 416). Hence, if a transformed cultured tobacco cell is prepared by introducing the ER gene into an ER-free tobacco cultured cell to express the ER and if the intracellular calcium ion concentration is changed by the addition of a glucan elicitor, the change is anticipated to trigger a resistance reaction by the glucan elicitor in plants other than soybean (e.g., tobacco), thereby allowing them to show resistance to a wide variety of fungi which use glucan as a mycelial wall component.

The change in intracellular Ca²⁺ concentration of transformed cultured tobacco cells by the addition of the elicitor was examined.

In this experiment, the transformed cultured tobacco cells (I 6) obtained by the kanamycin selection and the plasmid-containing cultured tobacco cells (C 2-4) were used.

The intracellular Ca²⁺ concentrations of the cultured cells were measured as follows with an acetoxymethyl derivative (Fura-2 AM) of a fluorescence chelator (Fura-2) for Ca²⁺ measurement:

Cells were harvested from about 2 ml of the transformed tobacco cell culture (corresponding to a cell volume of about 250 µl after standing for 10 minutes) by centrifugation (600 rpm, 30 seconds) and the supernatant was removed. To the cells was added 2 ml of a tobacco cultivation medium and the mixture was stirred gently and centrifuged (600 rpm, 30 seconds) to remove the supernatant. The same operations were repeated to wash the cultured cells. The washed cultured cells was suspended homogeneously in 2 ml of the medium. To 1 ml of the suspension of the cultured cells in the medium, 1 ml of the medium and 4 µl of 1 mM Fura-2 AM (final concentration: 2 µM, Dojin Chemical Co.) were added and the mixture was incubated in the dark for 30 minutes with occasionally stirring. Subsequently, the cells were washed 2 times with 2 ml of the medium by centrifugation (600 rpm, 30 seconds) to eliminate the free Fura-2 AM which was not incorporated into the cells. The washed cultured cells were suspended in 2 ml of the medium homogeneously and the suspension (2 ml) was transferred into a fluorescence-measurement cell. The incorporated Fura-2 AM should be changed to Fura-2 by hydrolysis with intracellular esterase. The fluorescence produced by the binding of Fura-2 to intracellular Ca²⁺ was measured at a fluorescence wavelength of 505 nm under exciting light of 335 nm with the cultured cells being stirred to ensure against precipitation of the cultured cells. The change in intracellular Ca²⁺ concentration was examined by measuring the fluorescence intensity at specified intervals of time after the addition of 50 µl of glucan elicitor (1 mg/ml) or deionized water to the cultured cells. For control, the change in intracellular Ca²⁺ concentration was examined on the plasmid-containing cultured tobacco cells by the same method as above. For another control, the change in intracellular Ca²⁺ concentration was examined on cultured soybean cells by the same method as above, except that the cultured cells were washed with a medium for soybean cells having the following formulation.
NaH₂PO₄·H₂O 75mg/ml, KH₂PO₄ 170mg/ml, KNO₃ 2,200mg/ml, NH₄NO₃ 600mg/ml, (NH₄ )₂SO₄ 67mg/ml, MgSO₄·7H₂O 310mg/ml, CaCl₂·2H₂O 295mg/ml, FeSO₄·7H₂O 28mg/ml, EDTA·Na₂ 37.3mg/ml, KI 0.75mg/ml, MnSO₄·4H₂O 10.0mg/ml, H₃BO₃ 3.0mg/ml, ZnSO₄·7H₂O 2mg/ml, Na₂MoO₄·2H₂O 0.25mg/ml, CuSO₄·5H₂O 0.025mg/ml, CoCl₂·6H₂O 0.025mg/ml, Inositol 100mg/ml, Nicotinic acid 1.0mg/ml, Pyridoxine·HCl 1.0mg/ml, Thiamine·HCl 10.0mg/ml, Glucose 5g/ml, Sucrose 25g/ml, Xylose 250mg/ml, Sodium pyruvate 5.0mg/ml, Citric acid 10.0mg/ml, Malic acid 10.0mg/ml, Fumaric acid 10.0mg/ml, N-Z-amine 500.0mg/ml, 2,4-dichlorophenoxyacetic acid 1.0mg/ml and Zeatine riboside 0.1mg/ml, adjusted to pH 5.7 with KOH.

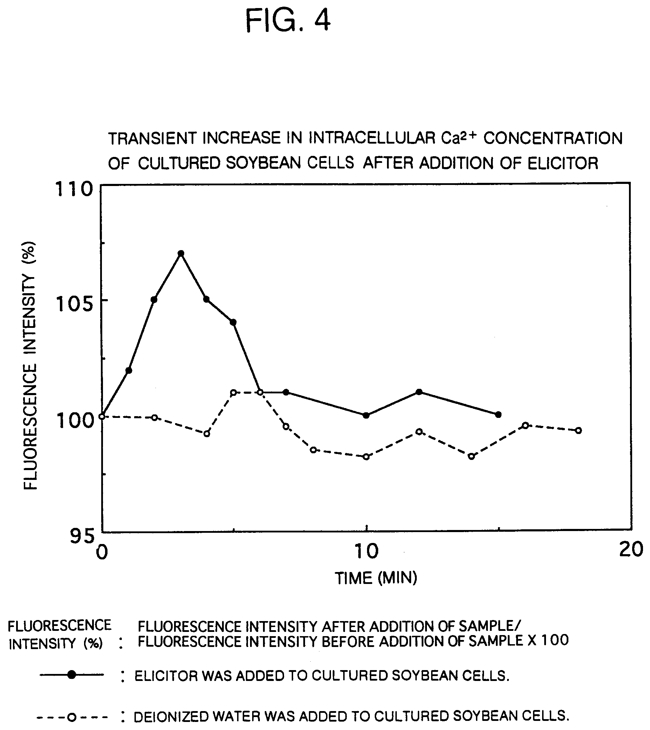

As a result of this experiment, about 7% transient increase in fluorescence intensity was observed in the cultured soybean cells 3 minutes after the addition of the elicitor, whereas no such change was observed after the addition of deionized water (Figure 4). The results suggest that the phenomenon in which the binding of the ER to the glucan elicitor caused a transient inflow of Ca²⁺ into cells could be observed in this experiment, thereby supporting the report that calcium ion plays an important role in the resistance reaction caused by the elicitor in cultured soybean cells. In the transformed cultured tobacco cells, about 10% transient increase in fluorescence intensity was observed 3 minutes after the addition of the elicitor, whereas no such change was observed after the addition of deionized water.

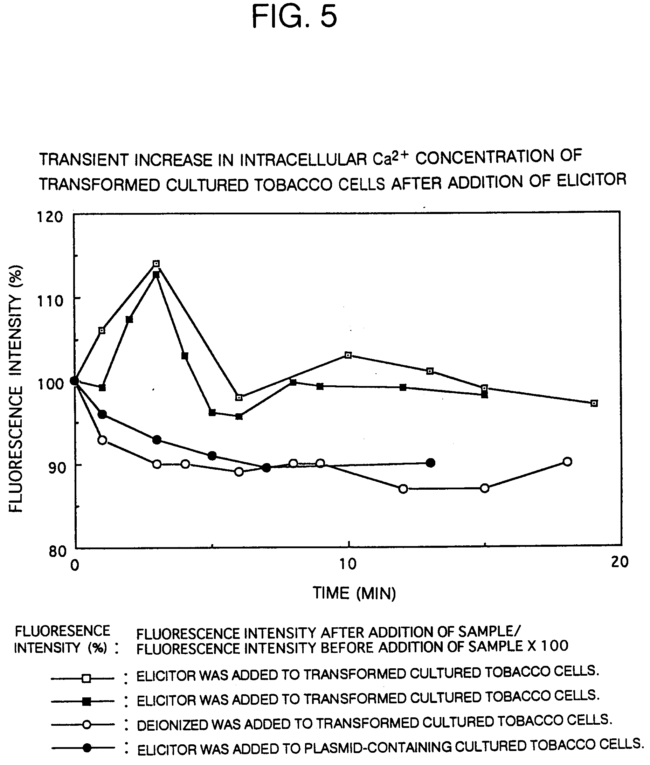

In the plasmid-containing cultured tobacco cells, none of the changes in fluorescence intensity that occurred in the transformed cultured tobacco cells was observed after the addition of the elicitor (Figure 5).

These results show that plants other than soybean (e.g., tobacco), which are not reactive with the glucan elicitor acquire the reactivity by introducing the gene of the soybean-derived glucan elicitor receptor for ER expression. Although the signal transduction pathway of each plant has not been completely explicated, it is expected that plants other than tobacco will acquire the reactivity with the glucan elicitor (i.e., a transient increase in intracellular Ca²⁺ concentration) by introducing the gene of the present ER for ER expression, thereby enabling the development of plants having resistance to a wide variety of fungi which use glucan as a mycelial wall component.

### Example 4

### Expression of Soybean ER in E. coli and Determination of Elicitor-binding Domain

### 1) Expression of Elicitor-binding Domain in E. coli

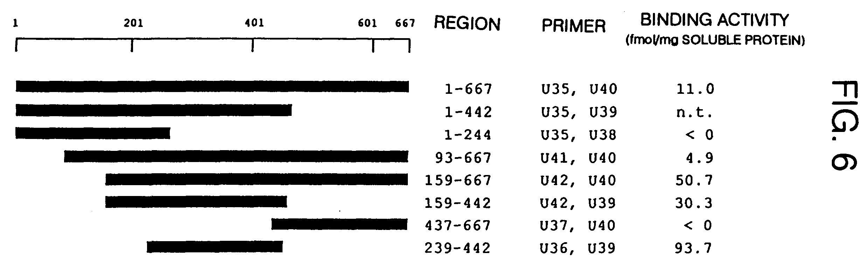

A fused protein of a partial fragment of the soybean ER with a maltose-binding protein (MBP) was prepared with a Protein Fusion & Purification System (New England Biolabs Co.) in order to express the partial fragment of the soybean ER in E. coli. PCR was performed using pER23-1 as a template to give DNA fragments of various lengths. The primers were designed to produce the MBP and fused protein in cloning into plasmid pMAL-c2 (New England Biolabs Co.) by adding a BamHI site on the 5' side and a SalI site on the 3' side exterior to the DNA molecule encoding the full-length portion and fragments of soybean ER shown in Figure 6. These primers were synthesized with an automatic nucleic acid synthesizer (Model 394 of Applied Biosystems Co.). The following primers were used in the amplification of the DNA chain.
Primer U35 5'-ATGGATCCATGGTTAACATCCAAACC-3' (SEQ ID NO:17);
Primer U36 5'-ATGGATCCGAATATAACTGGGAGAAG-3' (SEQ ID NO:18);
Primer U37 5'-ATGGATCCCCAGCATGGGGTAGGAAG-3' (SEQ ID NO:19);
Primer U38 5'-TAGTCGACTACTTCTCCCAGTTATATTC-3' (SEQ ID NO:20);
Primer U39 5'-TAGTCGACTACTTCCTACCCCATGCTGG-3' (SEQ ID NO:21);
Primer U40 5'-TAGTCGACTATTCATCACTTCTGCTATG-3' (SEQ ID NO:22);
Primer U41 5'-ATGGATCCGCCCCACAAGGTCCCAAA-3' (SEQ ID NO:23); and
Primer U42 5'-ATGGATCCAATGACTCCAACACCAAG-3' (SEQ ID NO:24)

The DNA molecule of pER23-1 (0.01 µg) was dissolved in 79 µl of distilled water. Either a combination of primers U5 and U7 or a combination of primers U10 and U12 (100 pmol each) and 0.5 µl of Taq DNA polymerase (TAKARA SHUZO CO., LTD.) were added to 8 µl of 2.5 mM dNTP in 10 µl of a 10 x PCR buffer (attached to Taq DNA polymerase of TAKARA SHUZO CO., LTD.) to give a final amount of 100 µl. PCR reaction was performed with a Gene Amp PCR System 9600 (Perkin-Elmer Co.) by 30 cycles of 1) denaturation at 94 °C x 30 seconds, 2) renaturation at 55°C x 30 seconds and 3) elongation at 72 °C x 1 minute. After the reaction, 15 µl of the reaction solution was digested with restriction enzymes BamHI and SalI and electrophoresed on a 1% agarose gel.

The gel was stained with a 0.5 µg/ml ethidium bromide solution for 15 minutes. The band showing the expected specific amplification was sectioned while observing under UV light. The gel section was treated with Gene Clean II (Bio101 Co.) to collect a DNA-containing solution. The collected DNA fragments were cloned into the BamHI-SalI site of plasmid pMAL-c2 and the clones were introduced into E. coli DH5α.

### 2) Preparation of Soluble Protein Fraction from E. coli

The E. coli cells into which the plasmids were introduced were precultured in an expression medium [10g/l tryptone (Gibco Co.), 5 g/l yeast extract (Gibco Co.), 5 g/l NaCl, 2 g/l glucose and 100 µg/ml ampicillin]. The precultured solution (0.4 ml) was added to 40 ml of the expression medium and cultured at 37 °C with shaking until OD₆₀₀ of 0.55 was reached. Isopropylthiogalactoside was added to the culture solution to give a final concentration of 0.3 mM and the shaking culture was continued for an additional 4 hours to induce expression. The E. coli was collected by centrifugation and the E. coli cells were washed with a washing buffer (20 mM Tris-HCl, pH 7.4, 200 mM NaCl and 1 mM EDTA). The cells were sonicated for a total of 2 minutes (15 sec x 8). ZW3-12 was added to the sonicated cells to give a final concentration of 0.25% and the mixture was incubated at 4 °C for 30 minutes. The supernatant was collected by centrifugation (10,000 rpm, 5 minutes) to give an E. coli soluble protein fraction. The expression of the fused protein was confirmed by an immunoblotting technique using an anti-maltose-binding protein antibody (New England Biolabs Co.).

### 3) Elicitor-binding Experiment

The elicitor-binding activity was determined as follows:

A complex of an elicitor and tyramine (TOKYO KASEI KOGYO CO., LTD.) was synthesized by the method of Jong-Joo Cheong (The Plant Cell (1991) 3: 127). The elicitor-tyramine complex was labelled with iodine-125 using chloramine T. The resulting sample (protein amount < 800 µg) was suspended in 500 µl of an assay buffer (50 mM Tris-HCl pH7.4, 0.1 M saccharose, 5 mM MgCl₂ , 1mM PMSF and 5 mM EDTA) and incubated at 0°C for 2 hours. The iodine-labelled elicitor-tyramine complex in an amount of 100 nM (70 Ci/mmol) was added to the suspension and the mixture was incubated at 4 °C for 2 hours. The reaction solution was filtered through Whatman GF/B (as treated with a 0.3% aqueous solution of polyethylenimine for at least 1 hour) and washed 3 times with 5 ml of an ice-cold buffer (10 mM Tris-HCl pH 7.0, 1 M NaCl, 10 mM MgCl₂). The radio activity retained on the filter membrane was counted with a gamma counter (count A). In order to eliminate the effect of non-specific binding, the same procedure as above was performed except that 17 µM of the elicitor was added to the same sample, the mixture was suspended in the assay buffer and the suspension was incubated at 0 °C for 2 hours. The obtained count was subtracted from the count A to give a count (Δ cpm) of elicitor-specific binding. The resulting count (Δ cpm) was divided by the total number of counts and then multiplied by the total amount of the elicitor used in the experiment to calculate the amount of the elicitor-binding protein (in moles).

As a result, a specific binding to the elicitor was observed in the E. coli transformed with the DNA molecule encoding the ER (Figure 6). Hence, it was reconfirmed that the obtained gene encoded a protein having the elicitor-binding activity and it was revealed that there was an elicitor-binding domain in the 239-442 amino acid sequence of SEQ ID NO:1.

### Example 5

### Inhibition of Binding of Glucan Elicitor to Elicitor-binding Protein in Soybean Cotyledon Membrane Fraction and Inhibition of Accumulation of Phytoalexin in Soybean Cotyledon by Antibody against Elicitor-binding Domain

### 1) Expression of Elicitor-binding Domain in E. coli

A fused protein of an elicitor-binding domain derived from the ER with a maltose-binding protein (MBP) was prepared with a Protein Fusion & Purification System (New England Biolabs Co.) in order to express a large amount of the elicitor-binding domain in E. coli. PCR was performed to produce a DNA molecule encoding the elicitor-binding domain. The following primers were synthesized with an automatic nucleic acid synthesizer (Model 394 of Applied Biosystems Co.):
Primer U36 5'-ATGGATCCGAATATAACTGGGAGAAG-3' (SEQ ID NO: 25); and
Primer U39 5'-TAGTCGACTACTTCCTACCCCATGCTGG-3' (SEQ ID NO: 26)

The DNA molecule of pER23-1 (0.01 µg) was dissolved in 79 µl of distilled water. Either a combination of primers U5 and U7 or a combination of primers U10 and U12 (100 pmol each) and 0.5 µl of Taq DNA polymerase (TAKARA SHUZO CO., LTD.) were added to 8 µl of 2.5 mM dNTP in 10 µl of a 10 x PCR buffer (attached to Taq DNA polymerase of TAKARA SHUZO CO., LTD.) to give a final amount of 100 µl. PCR was performed with a Gene Amp PCR System 9600 (Perkin-Elmer Co.) by 30 cycles of 1) denaturation at 94°C x 30 seconds, 2) renaturation at 55°C x 30 seconds and 3) elongation at 72 °C x 1 minute. After the reaction, 15 µl of the reaction solution was digested with restriction enzymes BamHI and SalI and electrophoresed on a 1% agarose gel.

The gel was stained with a 0.5 µg/ml ethidium bromide solution for 15 minutes. The band showing specific amplification was sectioned while observing under UV light. The gel section was treated with Gene Clean II (Bio101 Co.) to collect a DNA-containing solution. The collected DNA fragments were cloned into the BamHI-SalI site of plasmid pMAL-c2 and the clones were introduced into E. coli DH5α.

### 2) Purification of the Fused Protein Expressed in E. coli and Production of Antibody

The E. coli cells transformed with the plasmids were precultured in an expression medium (10g/l tryptone (Gibco Co.), 5 g/l yeast extract (Gibco Co.), 5 g/l NaCl, 2 g/l glucose and 100 µg/ml ampicillin) overnight. The precultured solution (150 ml) was added to 1.5 L of the expression medium and cultured in a Sakaguchi flask at 37°C with shaking until OD₆₀₀ of 0.55 was reached. Isopropylthiogalactoside was added to the culture solution to give a final concentration of 0.3 mM and the shakeculture was continued for an additional 4 hours to induce expression. The E. coli was collected by centrifugation and the E. coli cells were washed with a washing buffer (20 mM Tris-HCl, pH 7.4, 200 mM NaCl and 1 mM EDTA). The cells were sonicated for a total of 2 minutes (15 sec x 8). A soluble protein fraction was obtained by centrifugation. From this fraction, a MBP-fused protein was purified with an amylose resin. A MBP- and an elicitor-binding domain were cleaved with factor Xa and the elicitor-binding domain was purified by gel filtration column chromatography. The purified protein was injected twice into a mouse at the abdominal cavity for immunization by the method of E. Harlow and D. Lane (Antibody (1988) Cold Spring Harbor Co., pp. 53-137). After the increase in titer was confirmed by an ELISA method, the ascites was obtained and subjected to precipitation with 50% saturated ammonium sulfate and treated with Protein A Sepharose (Pharmacia Co.) to produce a purified antibody. In the treatment with Protein A Sepharose, the antibody was bound to Protein A Sepharose with 0.1 M sodium phosphate (pH 8.0) and eluted with 0.1 M citric acid (pH 3.5). It was confirmed by an immnoblotting that the obtained antibody recognized only the ER protein in soybean.

### 3) Preparation of Soybean Cotyledon Membrane Fraction

A soyben cotyledon membrane fraction was prepared as follows:

To soybean cotyledons cultured on soil for 9 days (wet weight: 36 g), 47 ml of an ice-cooled buffer (25 mM Tris-HCl, pH 7.0, 30 mM MgCl₂, 2 mM dithiothreitol, 2.5 mM sodium metabisulfite, 1 mM PMSF) was added and homogenized with a waring blender, followed by fractionation through centrifugation to form a precipitate of the cotyledon membrane fraction; the procedure was the same as in the preparation of the soybean root membrane fraction described in Section 2) of Example 1. The cotyledon membrane fraction was suspended in an ice-cooled buffer (10 mM Tris-HCl, pH 7.4, 0.1 M sucrose, 5 mM MgCl₂, 1 mM PMSF, 5 mM EDTA) and stored at -80 °C.

### 4) Measurement of Inhibition of Glucan Elicitor Binding to Elicitor-binding Protein of Soybean Cotyledon Membrane Fraction

The elicitor-binding activity was determined as follows:

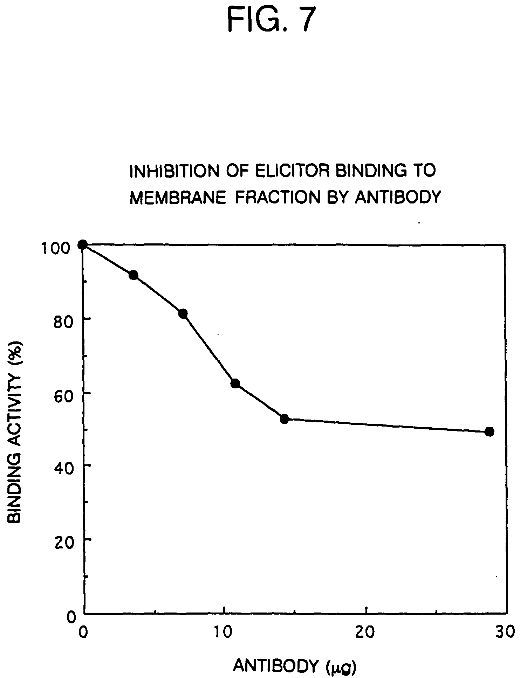

A complex of an elicitor and tyramine (TOKYO KASEI KOGYO CO., LTD.) was synthesized by the method of Jong-Joo Cheong (The Plant Cell (1991) 3: 127). The elicitor-tyramine complex was labelled with iodine-125 using chloramine T. The soybean cotyledon membrane faction (100 µl, 820 µg) was suspended in 500 µl of an assay buffer (50 mM Tris-HCl pH7.4, 0.1 M saccharose, 5 mM MgCl₂, 1mM PMSF and 5 mM EDTA) and incubated at 0°C for 2 hours. The iodine-labelled elicitor-tyramine complex in an amount of 714 ng (143 nM; 70 Ci/mmol) was added to the suspension and the mixture was incubated at 4 °C for 2 hours. The reaction solution was filtered through Whatman GF/B (as treated with a 0.3% aqueous solution of polyethylenimine for at least 1 hour) and washed 3 times with 5 ml of an ice-cold buffer (10 mM Tris-HCl pH 7.0, 1 M NaCl, 10 mM MgCl₂ ). The radio activity retained on the filter membrane was counted with a gamma counter (count A). In order to eliminate the effect of non-specific binding, the same procedure as above was performed, except that 100 times mole (75 µg, 15 µM) of a cold elicitor was added to the the sample, the mixture was suspended in the assay buffer and the suspension was incubated at 0°C for 2 hours. The obtained count was subtracted from the count A to give a count (Δ cpm) of elicitor-specific binding. Counts of binding obtained by adding 3.6, 7.1, 10.8, 14.4 and 28.8 µg of the purified antibody rather than the cold elicitor were subtracted from the count A. The resulting values were compared with that for the cold elicitor and expressed as the percentage, with the count (Δ cpm) of elicitor-specific binding being taken as 100% (Figure 7). The addition of 28.8 µg of the antibody resulted in the inhibition of the binding of elicitor by about 51%. The results confirmed that the antibody against the elicitor-binding domain inhibited the binding of the elicitor to the elicitor-binding protein.

### 5) Inhibition of Accumulation of Phytoalexin by Antibody against Elicitor-binding Domain

The amount of phytoalexin accumulated by the action of glucan elicitor was measured with soybean cotyledons by the method of M.G. Hahn et al. ((1992) Molecular Plant Pathology Volume II A Practical Approach, IRL Press, pp. 117-120).

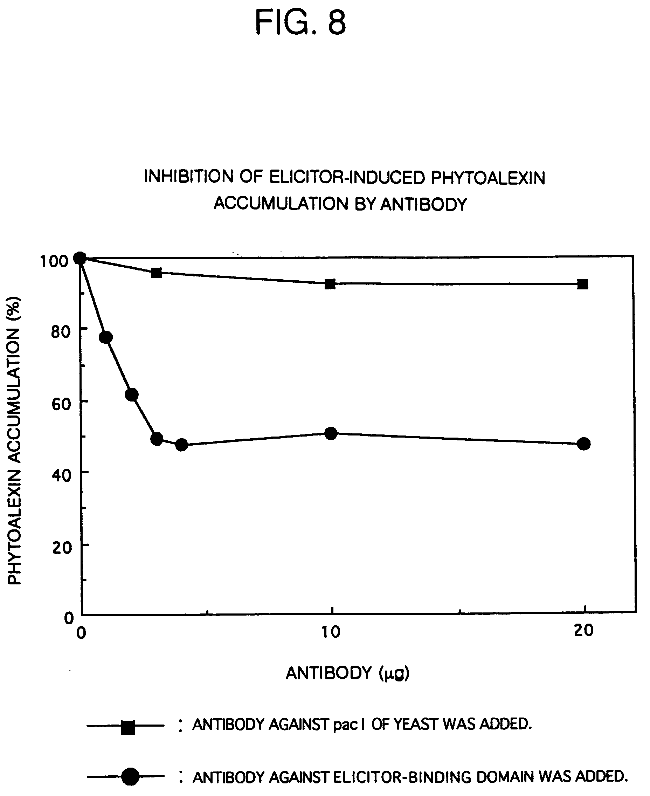

A purified antibody against the elicitor-binding domain (0, 1, 2, 3, 4, 10 and 20 µg/25 µl/cotyledon) or a purified antibody against yeast-derived dsRNAse, pac 1 (4, 10 and 20 µg/25 µl/cotyledon) as a control was added to soybean cotyledons and the mixture was incubated for 1 hour. Glucan elicitor (200 ng/25 µl/cotyledon) was added to the soybean cotyledons and the mixture was incubated for 20 hours to determine whether the accumulation of phytoalexin by the action of glucan elicitor was inhibited by the antibody. The amount of phytoalexin accumulation induced by the addition of elicitor subsequent to the addition of the antibody was expressed as the percentage, with the amount of phytoalexin accumulation by the sole addition of the elicitor being taken as 100% (Figure 8). When the antibody against the elicitor-binding domain was added in an amount of 20.0 µg per soybean cotyledon, the amount of phytoalexin accumulation decreased by about 53%. In the control, the amount of phytoalexin accumulation changed little even when the antibody against pac 1 was added in an amount of 20.0 µg per soybean cotyledon. These results showed that the obtained gene did not encode a mere elicitor-binding protein but encoded the ER inducing a resistance reaction in soybean.

### Example 6

### Transfer of ER Gene into Tobacco Plants

The soybean-derived ER gene was transferred into tobacco as described below, and the expression of the gene was confirmed.

### 1) Construction of Plant Expression Vector Plasmid

Plasmid pER23-1 is digested with BamHI and SalI to give an ER gene fragment sandwiched between the sites of the two restriction enzymes. This fragment is inserted into a plant vector to be described below. In a separate step, a plant expression-type binary plasmid pBI121 (Clonetech) was digested with restriction enzymes BamHI and SacI. Then, the following linker DNAs synthesized with an automatic nucleic acid synthesizer were annealed and ligated to the digested binary plasmid, which was introduced into E. coli DH5α . Desired plasmid pBIlinker was selected from the obtained clones.
5'-CTAGAGGATCCGGTACCCCCGGGGTCGACGAGCT-3' (SEQ ID NO: 27)
5'-CGTCGACCCCGGGGGTACCGGATCCT-3' (SEQ ID NO: 28)

The gene fragment described above was inserted between the cauliflower mosaic virus 35S promoter and the terminator of nopaline synthase (BamHI-SalI) in the resultant plasmid pBIlinker to produce a vector to be introduced into plants (pBI-ER).

### 2) Introduction of pBI-ER into Agrobacterium

Agrobacterium tumefaciens LBA4404 (Clonetech) was inoculated into 50 ml of YEB medium (containing 5 g of beef extract, 1 g of yeast extract, 1 g of peptone, 5 g of sucrose and 2 mM MgSO₄ per liter, pH 7.4) and cultured at 28°C for 24 hours. Then, the cells were harvested by centrifugation at 3,000 rpm and 4°C for 20 minutes. The cells were washed three times with 10 ml of 1 mM Hepes-KOH (pH 7.4), once with 3 ml of 10% glycerol and finally suspended in 3 ml of 10% glycerol to give an Agrobacterium into which DNA of interest was to be introduced.

Fifty µl of the thus obtained bacterium suspension and 1 µg of plasmid pBI-ER were placed in a cuvette, to which an electrical pulse was applied at 25 µF, 2500 V and 200 Ω using an electroporation apparatus (Gene Pulser; BioRad) to introduce the plasmid DNA into the Agrobacterium. The resultant solution was transferred to an Eppendorf tube, followed by the addition of 800 µl of SOC medium (containing 20 g of tryptone, 5 g of yeast extract, 0.5 g of NaCl, 2.5 mM KCl, 10 mM MgSO₄, 10 mM MgCl₂ and 20 mM glucose per liter, pH 7.0). The bacterium was subjected to stationary culture at 28 °C for 1.5 hours. Fifty µl of the resultant culture solution was plated on YEB agar medium (agar 1.2%) containing 100 ppm kanamycin and cultured at 28°C for 2 days.

A single colony was selected from the resultant colonies, and plasmid DNA was prepared from that colony by an alkaline method. This plasmid DNA was digested with an appropriate restriction enzyme, and the resultant DNA fragments were fractionated by 1% agarose gel electrophoresis and analyzed. As a result, it was confirmed that the plasmid DNA contained plasmid pBI-ER. This Agrobacterium tumefaciens is designated Agro-ER.

### 3) Transformation of Tobacco

The Agro-ER strain described above was cultured under shaking in LB liquid medium containing 50 ppm kanamycin at 28°C for 2 hours. Cells were harvested by centrifuging 1.5 ml of the culture solution at 10,000 rpm for 3 minutes and washed with 1 ml of LB medium to remove the kanamycin. Then, the cells were harvested by further centrifugation at 10,000 rpm for 3 minutes and re-suspended in 1.5 ml of LB medium to give a bacterium suspension for infection.

In infecting a tobacco variety Bright Yellow with the bacterium, young leaves were collected from a germ-free plant. These leaves were aseptically cut into pieces 1 cm² in size with a surgical knife, placed on the Agrobacterium suspension with back of each leaf facing up, and shaken gently for 2 minutes. Thereafter, the leaf pieces were placed on a sterilized filter paper to remove excessive Agrobacterium. Whatman No. 1 filter paper (φ 7.0 cm) was placed on MS-B5 medium (containing 1.0 ppm benzyladenine, 0.1 ppm naphthaleneacetic acid and 0.8% agar) (Murashige, T. and Skoog, F. Plant Physiol., 15: 473, (1962)) in a culture dish. The leaf pieces were placed upon this filter paper with the back of each leaf facing up. The culture dish was sealed with a PARAFILM (American National Can), and then the leaf pieces were cultured at 25°C for 2 days through cycles of 16 hours under light and 8 hours in the dark. Subsequently, the leaf pieces were transferred onto MS-B5 medium containing 250 ppm claforan and cultured in the same manner for another 10 days in order to remove the Agrobacterium. The leaf pieces were further transferred onto MS-B5 medium containing 250 ppm claforan and 100 ppm kanamycin and cultured in the same manner for another 7 days. During this period, the regions surrounding the leaf pieces changed to callus, yielding shoot primordia. After culturing for another 10 days, elongated shoots were placed on MS-HF medium (benzyladenine- and naphthaleneacetic acid-free MS-B5 medium) containing 250 ppm claforan and 100 ppm kanamycin. After culturing for 10 days, those shoots which were rooting were placed on MS-HF medium containing 250 ppm claforan in a plant box as kanamycin resistant transformants.

### 4) PCR and Immunoblot Analysis of Genomic DNA from the Transformant Tobacco

In order to confirm that the gene of interest was transferred into the transformant, a PCR was performed. The following primers were synthesized with an automatic nucleic acid synthesizer (Applied Biosystems; Model 394) and used in the PCR.
Primer ER1 5'-CACCTTCAGCAACAATGGTT-3' (SEQ ID NO: 29)
Primer ER2 5'-CTATTCATCACTTCTGCTAT-3' (SEQ ID NO: 30)

DNA was extracted from the kanamycin resistant transformant tobacco and examined. Genomic DNA was extracted as described below. Briefly, 20 mg of tobacco leaves was crushed with a plastic bar in 200 µl of an extraction buffer (0.5 M NaCl, 50 mM Tris-HCl, pH 8, 50 mM EDTA). Then, 60 µl of 20% polyvinyl pyrrolidone (mean molecular weight: 40 kDa) and 52 µl of 10% SDS were added and heated at 65°C for 30 minutes. Subsequently, 40 µl of 5 M potassium acetate was added, and the resultant mixture was left on ice for 30 minutes. Then, the mixture was centrifuged to recover the supernatant; 180 µl of isopropyl alcohol was then added to recover the DNA as a precipitate. After washing with 70% ethanol, the DNA was dissolved in 150 µl of TE solution (10 mM Tris-HCl, pH 8, 1 mM EDTA, 1 µg/ml RNase A). To 79 µl of distilled water, 1 µl of this DNA solution, 10 µl of 10x PCR buffer (attachment to Taq DNA polymerase; Takara Shuzo), 8 µl of 2.5 mM dNTP, 100 pmol each of primers ER1 and ER2, and 0.5 µl of Tag DNA polymerase (Takara Shuzo) were added to make a 100 µl solution. With this solution, a PCR was performed as follows. As a reaction apparatus, Gene Amp PCR System 9600 (Perkin-Elmer) was used. First, denaturation reaction was performed at 94 °C for 5 minutes. Then, 30 cycles of 1) denaturation at 94°C for 30 seconds, 2) renaturation at 55°C for 30 seconds and 3) extension at 72 °C for 1 minute were performed. After the reaction, 15 µl of the reaction solution was electrophoresed on 1% agarose gel. The gel was stained with a 0.5 µg/ml ethidium bromide solution for 15 minutes and examined under UV light. By confirming a specific DNA fragment of about 2 kbp which was expected to be amplified, it was confirmed that the gene of interest has been incorporated into the tobacco genomic DNA.

Immunoblot analysis was also performed to check for the expression of the gene of interest. Briefly, 20 mg of tobacco leaves was crushed with a plastic bar in 100 µl of an ice-cooled extraction buffer (0.1 M Tris-HCl, pH 7.5, 1 mM PMSF). Then, 50 µl of 3x SDS-PAGE sample buffer (30% glycerol, 3% β -mercaptoethanol, 3% SDS, 0.19 M Tris-HCl, pH 6.8, 0.001% BPB) was added and heated at 100°C for 5 minutes. The resultant mixture was centrifuged at 12,000 rpm for 5 minutes to recover the supernatant. A portion (15 µl) of the extracted protein was subjected to SDS-polyacrylamide gel electrophoresis and transferred onto a PVDF membrane (Millipore). Immunoblotting was performed on this membrane using the anti-ER mouse antibody prepared in Example 5 as a primary antibody, anti-mouse immunoglobulin alkaline phosphatase-labeled antibody (Jackson) as a secondary antibody and also an alkaline phosphatase coloring substrate (Wako Pure Chemical Industries) to assay the expression of the ER protein. There were a plurality of plants expressing various amounts of the ER protein. From these plants, those expressing a large quantity of the ER protein were selected and subjected to a hypersensitive reaction test and a fungus resistance test.

### Example 7

### Hypersensitive Reaction Test of the Tobacco Transformant

Induction of hypersensitive reaction by a soybean elicitor was examined using leaves of the tobacco transformants in which high expression of the ER protein had been confirmed in Example 6, as well as leaves of non-transformed tobacco plants and those tobacco plants transformed with the vector (pBI121) alone as controls.

Leaves of tobacco plants grown in a green house were cut off at the petiole and placed in a light-transmissive plastic box. A piece of silicone tube 5 mm in diameter and 5 mm in height was put on the upper surface of each leaf so that a solution could be retained. This tube piece was allowed to retain a chemically synthesized elicitor (β-D-glucohexaoside) [N. Hong and T. Ogawa (1990), Tetrahedron Lett. 31: 3179; released from Prof. Ogawa of the Institute of Physical and Chemical Research and the University of Tokyo] dissolved in a buffer solution (3 mM sodium bicarbonate, 4 mM sodium acetate, pH 8.0) or the buffer solution alone such that the solution was kept in contact with the surface of each leaf. The leaves were cultured under excessive moisture to prevent drying of the solution through cycles of 16 hours under light and 8 hours in the dark at 25 °C for 7 day. After the culture, the silicone tube was removed, and the induction of synthesis of tobacco phytoalexin was examined on a UV illuminator (Funakoshi). Nothing could be found from the combination of non-transformed tobacco and the chemically synthesized glucan elicitor or from the combination of tobacco plants transformed with the vector alone and the glucan elicitor. This means that tobacco cannot recognize this glucan elicitor. On the other hand, from the combination of tobacco TF1-11-1-15 transformed with the ER gene and the chemically synthesized glucan elicitor, accumulation of a remarkable amount of a fluorescent substance (phytoalexin) was recognized. Also, no changes were observed in controls, i.e., those combinations of the tobacco plants and the buffer solution alone or deionized water.

From these results, it was proved that, if the ER gene can be expressed in a host plant other than soybean, there is a possibility that the transformed host plant may acquire the ability to recognize a glucan elicitor which the non-transformed host cannot recognize. The present invention provides not only a possibility to change the plant recognition of a substance, but also a mechanism by which a plant can recognize plant pathogens such as fungi having a glucan structure in their cell walls or the like. As a result, resistant reactions such as induction of phytoalexin closely involved in disease resistance is elicited in the plant. Elicitation of such resistant reactions is important for breeding disease resistant plants.

### Example 8

### Fungus Resistance Test of the Tobacco Transformant

The tobacco transformants in which high expression of the ER protein had been confirmed in Example 6 were selfed or crossed with glucanase-expressing tobacco plants (Japanese Unexamined Patent Publication No. 4-320631) to harvest seeds, the subsequent generation.

### 1) Resistance to Phytophthora nicotianae

A strain conserved at Hokkaido University (subcultured in PDA medium from Difco) was transferred to an oatmeal agar and cultured at 26 °C for 4 days. The growing end portion of the mycelium spreading all over the medium was punched with a cork borer to form mycelium disks, which were used as an inoculant. The oatmeal agar medium used in this experiment was prepared as follows. One hundred grams of oatmeal powder was suspended in 1 liter of water, heated at 58 °C for 1 hour and filtered with gauze. To the filtrate, 20 g of agar was added and autoclave-sterilized. Then, the resultant mixture was dispensed into culture dishes for use as a medium.

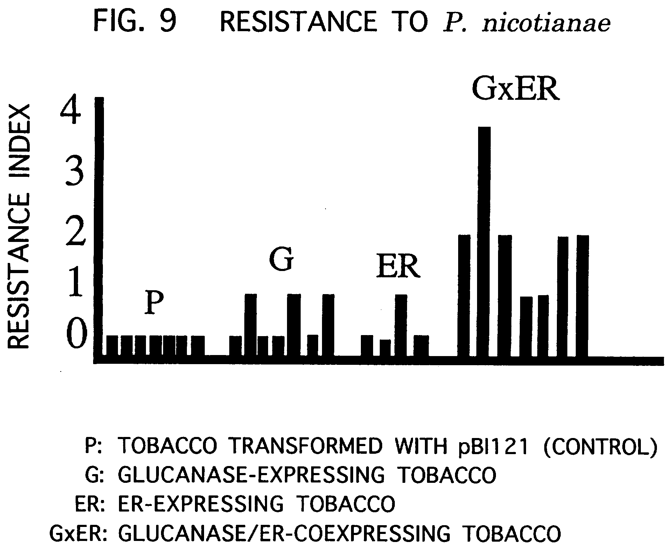

Seedlings obtained from the above-mentioned seeds were tested for expression of ER based on the method described in Example 6. Then, the fungus was inoculated into wounds of those seedlings in which a remarkable expression of ER had been confirmed. Briefly, leaves cut off from tobacco plants about 2 months after germination were placed on a moisturized filter paper in a plastic box. Ten needles tied up together were applied 30 times at one point on both the right and the left side in each of the leaves, yielding punctured wounds in the form of a concentric circle. A small amount of deionized water was applied to the wounds, and then the mycelium disk was inoculated into each wound. Thereafter, the leaves were left at 25 °C for 96 hours. The results of this test are shown in Fig. 9 and Table 3. The resistance of each tobacco leaf tested is shown with a resistance index. The resistance indexes are as follows. "4": no disease symptom; "3": up to 25% of the surface bears disease symptoms on both sides of the leaf; "2": up to 50% of the surface bears disease symptoms on both sides of the leaf; "1": up to 75% of the surface bears disease symptoms on both sides of the leaf; "0": 75% or more of the surface bears disease symptoms on both sides of the leaf.

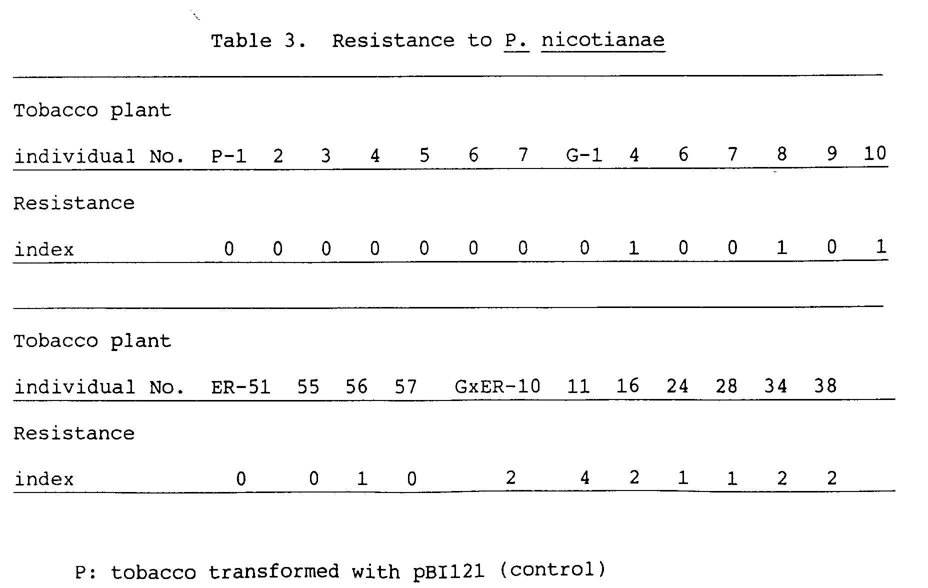

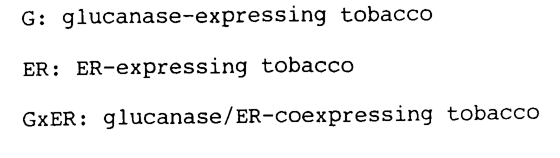

As a result, the formation and expansion of lesions were observed in control plants (plants transformed with pBI121 and plants expressing glucanase alone) whereas the expansion of lesions was not so remarkable in most of the transformants coexpressing glucanase and ER. Therefore, it is believed that the resistance to fungi was improved by the transfer of the ER gene.

### 2) Resistance to Rhizoctonia solani

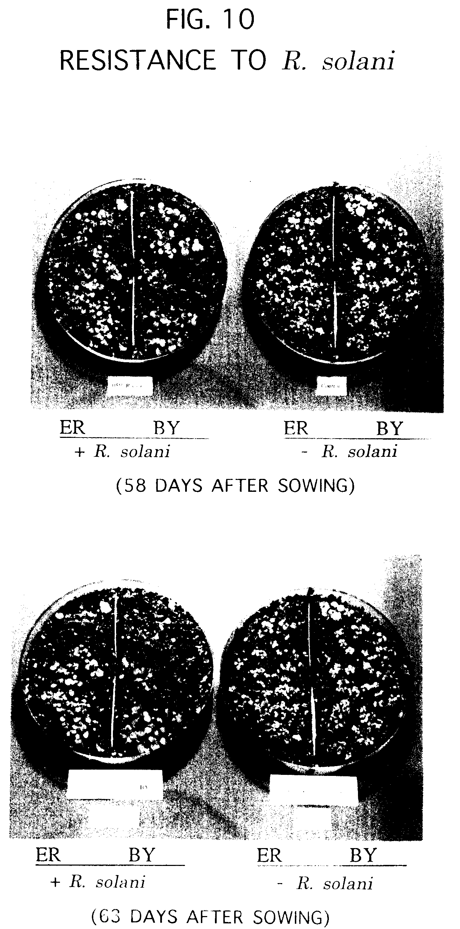

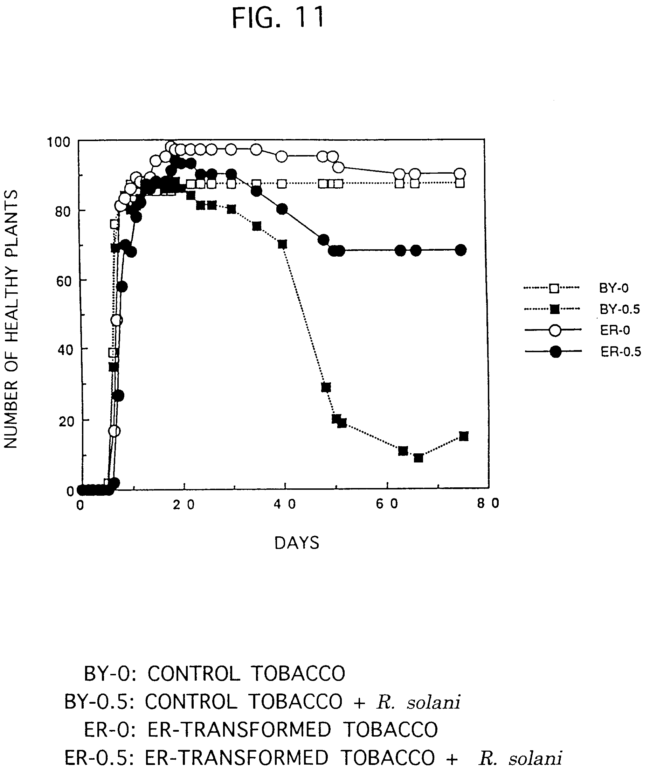

A strain conserved at Gifu University (Rhizoctonia solani AG3 M strain; released from Prof. Hyakumachi of Gifu University; subcultured in PDA medium from Difco) was inoculated into an autoclave-sterilized mixture of barley grains and deionised water (50:50 by volume), cultured at 24°C for 10 days and dried for 10 days. Then, the barley grains were milled by a coffee maker and mixed well with a soil (river sand: vermiculite: peat moss = 2:2:1) at a ratio of 0.5% (w/w). The test seeds were sown on this mixture and grown through cycles of 16 hours under light and 8 hour in the dark at 25 °C under a humidity of 60-80%. Their growth was observed and, finally, the number of healthy individuals for each of the tested tobacco plants was counted (Figs. 10 and 11).

As a result, the formation and expansion of lesions were observed in control plants (non-transformed tobacco); the number of healthy individuals decreased sharply; and most of the individuals withered. On the other hand, in ER-expressing transformants, lesions were hardly observed or delay in the development of disease symptoms was observed (Fig. 10). Therefore, it is believed that the resistance to fungi was improved by the transfer of the ER gene.

### 3) Fungus Resistance Test using Zoospores of Phytophthora nicotianae

In addition to the fungus resistance test by needle inoculation described in section 1) of Example 8, another fungus resistance test was conducted by inoculating a zoospore suspension. A fungus strain conserved at Hokkaido University (subcultured in PDA medium from Difco) was transferred to an oatmeal agar and cultured at 25°C in the dark for 1 week. The oatmeal agar was prepared by suspending 100 g of oatmeal powder in 1 liter of water, heating at 58°C for 1 hour and filtering with gauze; to the filtrate, 20 g of agar was added, autoclave-sterilized and dispensed into culture dishes for use as a medium. From the resultant mycelial flora, disks were punched with a cork borer 6 mm in diameter. The disks were placed on 9 cm plastic culture dishes at regular spacings (7 disks/dish). To each dish, 25 ml of a soybean decoction medium (obtained by grinding 400 g of green soybean, filtering the resultant material with gauze and adding distilled water to the filtrate to make a 1 liter solution, followed by autoclave sterilization) was added, and the disks were cultured at 25 °C in the dark for 3 days. After confirming that mycelial mat was formed on almost all over the culture dishes, the medium was discarded. The mycelial mat was washed with an aqueous Petri solution (1 mM KCl, 2 mM Ca(NO₃ )₂ , 1.2 mM MgSO₄ , 1 mM KH₂PO₄ ) three or four times to remove the medium components as completely as possible. Finally, the mycelial mat was washed once with a soil extract (prepared by adding water to 11.5 g of field soil to make a volume of 1 liter, filtering the mixture and sterilizing in an autoclave). After swishing water off, the mycelial mat was left at 15 °C under lighting for several days until its surface became slightly dry. It has been found for the first time that this drying treatment is very important for the formation of a large quantity of zoosporangia of the fungus. To the thus formed zoosporangia, the soil extract was added and left to stand at 15 °C under lighting for 2-3 hours. After confirming that a sufficient amount of zoospores was formed, the zoospores were collected to give an inoculant.

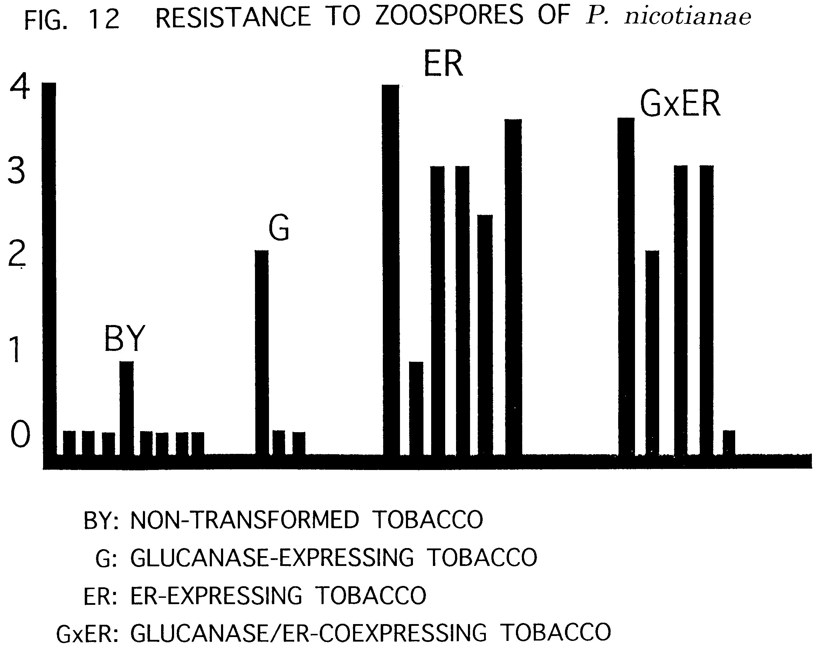

The zoospores were inoculated to those plants in which a remarkable expression of ER had been confirmed based on the method described in Example 6. Briefly, leaves of tobacco plants about 4 months after germination were cut off and placed on a moisturized filter paper in a plastic box. A silicone ring cut into about 5 mm in length was placed on both the right and the left side of each of the leaves. Then, 100 µl of a zoospore suspension (3-5 x 10⁵ zoospores/ml) was added into the silicone ring with a micropipette to thereby inoculate the zoospores to the surface of the tobacco leaf. Then, each leaf was left at 25°C for 144 hours. The results of this test are shown in Fig. 12 and Table 4.

The resistance of each tobacco leaf tested is shown with a resistance index. The resistance indexes are as follows. "4": no disease symptom; "3.5": lesions are restricted to the inoculation site; "3": up to 25% of the half leaf bears disease symptoms; "2.5": up to 37.5% of the half leaf bears disease symptoms; "2": up to 50% of the half leaf bears disease symptoms; "1": up to 75% of the half leaf bears disease symptoms; "0": more than 75% of the half leaf bears disease symptoms.

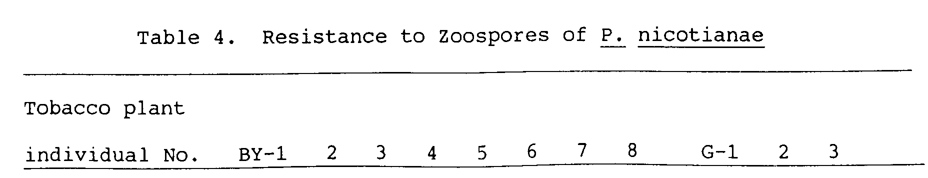

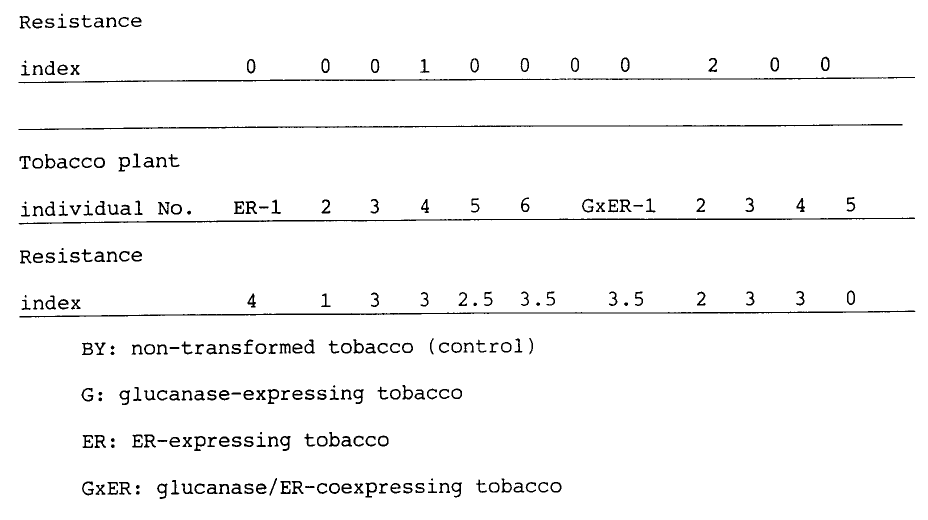

As a result, the formation and expansion of lesions were observed in control plants (non-transformed tobacco and tobacco expressing glucanase alone) whereas the expansion of lesions were inhibited in most of the transformants expressing ER alone or ER and glucanase. Therefore, it is believed that the resistance to fungi was improved by the transfer of the ER gene.

### Example 9

### Cloning of Novel Kidney Bean Glucanase

### 1) Preparation of Soybean mRNA

Kidney bean (Hirasaya Fancy Saitou) seeds (Takayama Seed Co.) were cultured on vermiculite for 12 days and then treated with ethylene for 48 hours according to the method of U. Vogeli et al. [Planta (1988) 174: 364] in order to induce the expression of glucanase. The plants were frozen in liquid nitrogen and stored at -80 °C until use. According to the method of Ishida et al. ("Cell Technology Laboratory Manipulation Manual" authored by Ishida and Misawa, Kodansha Scientific), 2.35 mg of total RNA was obtained from 12 g of frozen kidney bean powder.

Subsequently, 1.0 mg of the thus obtained total RNA was used for purification with Oligo (dT) Cellulose (Pharmacia) according to the manual and then 31.5 µg of purified poly(A)+RNA was obtained.

### 2) Preparation of Kidney Bean cDNA Library

cDNA was synthesized from 5 µg of the poly(A)+RNA with Time Saver cDNA Synthesis Kit (Pharmacia) and random hexamer primers. The synthesized cDNA fragments were ligated to lambda phage vector λ gt10 (Stratagene) with T4 DNA ligase (Takara Shuzo). Subsequently, the phage vectors were packaged to form phage particles with Gigapack (Stratagene) using a DNA mixed solution to thereby prepare a kidney bean cDNA library of about 1 x 10⁵ pfu.

### 3) Preparation of a Screening Probe

Based on the report of B.V. Edington et al. [Plant Molecular Biology (1991) 16:81] on the cloning of kidney bean glucanase cDNA, PCR primers were prepared as follows: sense primer: 5'-CAAATGTTGTGGTGAGGGATG GCC-3' (SEQ ID NO: 31) and antisense primer: 5'-AAATGTTTCTCTATCTCAGGACTC -3' (SEQ ID NO: 32). An RT-PCR was performed with these primers according to the method of Ishida ("Gene High Expression Experiment Manual", Ishida and Ando (Eds.), Kodansha Scientific) to give a PCR fragment of about 300 bp. This fragment was subcloned into the EcoRV site of pBluescriptSKII+ (Stratagene). For the cDNA synthesis, 1 mg of total RNA and 0.5 mg of random hexamer primers (Takara Shuzo) were used. The DNA sequence of the insert (0.3 kbp) in the subclone plasmid was determined. As a result, the DNA sequence was found to be identical with the glucanase cDNA reported by B.V. Edington et al.(supra). This plasmid DNA was digested with HindIII and EcoRV, and fractionated by agarose gel electrophoresis. The insert DNA was purified with Gene Clean II (Bio 101) to give a probe for screening the kidney bean cDNA library prepared in 2) above.

### 4) Screening and Cloning of the Library by Hybridization

The DNA fragment obtained as a screening probe was labelled with [α-³²P]dCTP using Megaprime DNA labelling kit (Amersham) according to the manual, and the reaction solution was subjected to the subsequent hybridization experiment.

E. coli C600 hf1 (Invitrogen) was transfected with the kidney bean cDNA library prepared in 2) above, and inoculated into L medium supplemented with 10 mg/ml MgCl₂ in a culture dish about 15 cm in diameter to form a total of about 1 x 10⁵ plaques. The plaques were blotted to a nylon membrane (GeneScreen(+); NEN DuPont). The membrane was reacted with the ³²P-dCTP-labelled glucanase cDNA fragment, and positive phages detected by autoradiography were screened again in the same manner to give one phage clone.

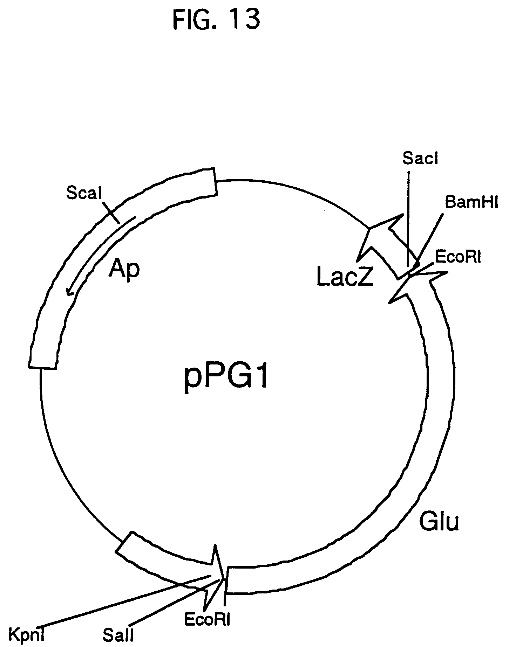

The λ phage DNA was purified with Lambda Sorb (Promega) from a solution of the positive clone isolated in the hybridization experiment. Five micrograms of this DNA was digested with EcoRI and fractionated by 1% agarose gel electrophoresis to cut out an about 1.2 kb band. This band was treated with Gene Clean II (Bio 101) to recover a solution containing the DNA, which was subcloned into the EcoRI site of vector pBluescriptII KS+ (Stratagene). Fig. 13 shows the structure of plasmid pPG1.

### 5) Determination of the Nucleotide Sequence of the DNA Coding for Kidney Bean Glucanase

The DNA of the plasmid into which the glucanase cDNA had been cloned was sequenced in both orientations with a fluorescence sequencer by preparing a series of plasmids having deletions at intervals of ca. 200-300 bp, using Kilosequence Deletion Kit (Takara Shuzo). The resultant nucleotide sequence for the DNA is shown in SEQ ID NO: 33. As a result, the DNA fragment was found to contain an ORF of 993 bp starting from a nucleotide sequence corresponding to an amino acid sequence that was predictably to be a signal sequence; it is presumed that 331 amino acid residues are encoded in the ORF. This amino acid sequence is shown in SEQ ID NO: 34.

As a result of a search using BLAST Protein Search, the amino acid sequence deduced from the resultant nucleotide sequence was found to be a completely novel sequence. Compared with the amino acid sequence reported previously by B.V. Edington et al. [Plant Molecular Biology (1991) 16:81], there was 49% homology (excluding the portion which appeared to be a signal sequence). Besides, it had 51% homology in full length to the soybean-derived amino acid sequence reported by Y. Takeuchi et al. [Plant Physiol. (1990) 93:673]. Since the deduced amino acid sequence exhibits high homology to the amino acid sequences of the previously reported glucanases, it was expected that the resultant DNA sequence would also encode a glucanase. It was also expected that, unlike previously reported kidney bean glucanases, the glucanase under discussion was of an extracellular secreting type since the deduced amino acid sequence had a would-be signal sequence at its N-terminal while lacking a would-be vacuole targeting sequence at its C-terminal.

### Example 10

### Expression of the Kidney Bean Glucanase

### 1) Construction of Plasmid pGST-PG1

PCR sense primer: 5'-GGAATTCCGAATCTGTGGGTGTGTGTTAT-3' (SEQ ID NO: 35) and antisense primer: M13 reverse sequence primer (SEQ ID NO: 36) were designed so that the kidney bean glucanase sequence [excluding the signal sequence, i.e. Met(1)-Val(21) at the N-terminal] could be ligated downstream from glutathione-S-transferase expression vector (Pharmacia; pGEX-4T-3) in an in-frame fashion. With these primers, a PCR was performed on 0.1 mg of a template plasmid pPG1 DNA using EX-Taq DNA polymerase (Takara Shuzo) (annealing temperature = 50°C ; 20 cycles). The amplified PCR fragments were digested with EcoRI and fractionated by agarose gel electrophoresis to give a DNA fragment of about 1 kbp. This fragment was purified with Gene Clean II and subcloned into the EcoRI site of pGEX-4T-3 expression vector using JM109 competent cells (Toyobo) (pGST-PG1).

### 2) Expression in E. coli BL21

The plasmid DNA was purified from the subclone obtained in 1) above and re-transferred into E. coli BL21 competent cells (Molecular Cloning, Cold Spring Harbor Laboratory Press). The E. coli BL21 was released from Prof. Masayuki Yamamoto, the Department of Science, the University of Tokyo.

### 3) Purification of GST-Fused Glucanase

An overnight culture (4 ml) of the E. coli obtained in 1) above was transferred into 200 ml of 2 x YT medium containing 100 mg/ml ampicillin and cultured at 37 °C for 1.5 hours, IPTG (Takara Shuzo) was added thereto to give a final concentration of 0.1 mM, and the cells were cultured for another 4 hours. The cells were harvested from the culture solution by centrifugation at 10,000 rpm and 4°C for 10 minutes.

According to the Gene Expression Experiment Manual (supra), approximately 1 mg of glutathione-S-transferase/glucanase fusion protein (molecular weight: about 62 kDa) was purified using Glutathione Sepharose (Pharmacia).

### 4) Determination of Glucanase Activity

The glucanase activity of the purified glutathione-S-transferase/glucanase fusion protein was determined by the following procedure. Briefly, an enzymic reaction solution was incubated at 37°C . The reaction was terminated at 0, 10, 20 and 30 minutes from the start of the reaction, and the liberated glucose was quantitatively determined by the method of Nelson [N. Nelson, J. Biol. Chem. (1944) 153, 375]. The enzymic reaction solution was composed of 0.5 ml of 50 mM acetate buffer (pH 5.5), 2.5 mg of laminarin as a substrate, and 0, 0.51 or 5.1 µg of the glutathione-S-transferase/glucanase fusion protein as an enzyme .

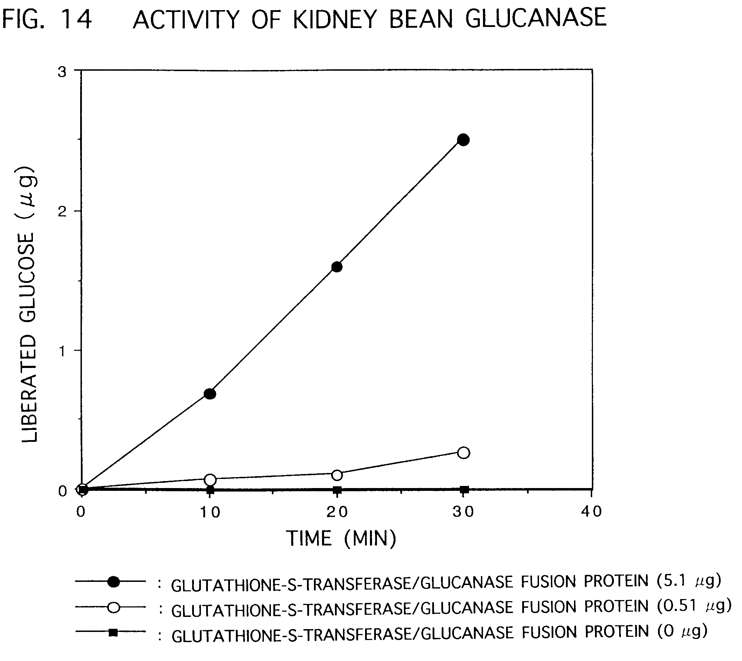

As a result,, glucose was liberated from laminarin in a manner dependent on both enzyme concentration and reaction time (see Fig. 14). Thus, it was made clear that the newly cloned cDNA has glucanase activity. This suggests the possibility that the glucanase under consideration can also be utilized for improving plants' resistance to fungi, like the soybean-derived glucanase encoded by SEQ ID NO: 4.

### INDUSTRIAL APPLICABILITY

According to the present invention, a plant into which a DNA sequence coding for a glucan elicitor receptor has been transferred and which expresses the glucan elicitor receptor, as well as a method for creating the plant are provided.

The plant of the present invention has high resistance to fungi.

Sequence Listing

INFORMATION FOR SEQ ID NO:1

LENGTH: 667 amino acids

TYPE: amino acid

TOPOLOGY: linear

MOLECULE TYPE: peptide

SEQUENCE DESCRIPTION: SEQ ID NO:1:

Val Asn Ile Gln Thr Asn Thr Ser Tyr Ile Phe Pro Gln Thr Gln Ser Thr Val

1               5                   10                  15

Leu Pro Asp Pro Ser Lys Phe Phe Ser Ser Asn Leu Leu Ser Ser Pro Leu Pro

    20                  25                  30                  35

Thr Asn Ser Phe Phe Gln Asn Phe Val Leu Lys Asn Gly Asp Gln Gln Glu Tyr

            40                  45                  50

Ile His Pro Tyr Leu Ile Lys Ser Ser Asn Ser Ser Leu Ser Leu Ser Tyr Pro

55                  60                  65                  70

Ser Arg Gln Ala Ser Ser Ala Val Ile Phe Gln Val Phe Asn Pro Asp Leu Thr

        75                  80                  85                  90

Ile Ser Ala Pro Gln Gly Pro Lys Gln Gly Pro Pro Gly Lys His Leu Ile Ser

                95                  100                 105

Ser Tyr Ser Asp Leu Ser Val Thr Leu Asp Phe Pro Ser Ser Asn Leu Ser Phe

    110                 115                 120                 125

Phe Leu Val Arg Gly Ser Pro Tyr Leu Thr Val Ser Val Thr Gln Pro Thr Pro

            130                 135                 140

Leu Ser Ile Thr Thr Ile His Ser Ile Leu Ser Phe Ser Ser Asn Asp Ser Asn

145                 150                 155                 160

Thr Lys Tyr Thr Phe Gln Phe Asn Asn Gly Gln Thr Trp Leu Leu Tyr Ala Thr

        165                 170                 175                 180

Ser Pro Ile Lys Leu Asn His Thr Leu Ser Glu Ile Thr Ser Asn Ala Phe Ser

                185                 190                 195

Gly Ile Ile Arg Ile Ala Leu Leu Pro Asp Ser Asp Ser Lys His Glu Ala Val
    200                 205                 210                 215
Leu Asp Lys Tyr Ser Ser Cys Tyr Pro Val Ser Gly Lys Ala Val Phe Arg Glu
            220                 225                 230
Pro Phe Cys Val Glu Tyr Asn Trp Glu Lys Lys Asp Ser Gly Asp Leu Leu Leu
235                 240                 245                 250
Leu Ala His Pro Leu His Val Gln Leu Leu Arg Asn Gly Asp Asn Asp Val Lys
        255                 260                 265                 270
Ile Leu Glu Asp Leu Lys Tyr Lys Ser Ile Asp Gly Asp Leu Val Gly Val Val
                275                 280                 285
Gly Asp Ser Trp Val Leu Lys Thr Asp Pro Leu Phe Val Thr Trp His Ser Ile
    290                 295                 300                 305
Lys Gly Ile Lys Glu Glu Ser His Asp Glu Ile Val Ser Ala Leu Ser Lys Asp
            310                 315                 320
Val Glu Ser Leu Asp Ser Ser Ser Ile Thr Thr Thr Glu Ser Tyr Phe Tyr Gly
325                 330                 335                 340
Lys Leu Ile Ala Arg Ala Ala Arg Leu Val Leu Ile Ala Glu Glu Leu Asn Tyr
        345                 350                 355                 360
Pro Asp Val Ile Pro Lys Val Arg Asn Phe Leu Lys Glu Thr Ile Glu Pro Trp
                365                 370                 375
Leu Glu Gly Thr Phe Ser Gly Asn Gly Phe Leu His Asp Glu Lys Trp Gly Gly
    380                 385                 390                 395
Ile Ile Thr Gln Lys Gly Ser Thr Asp Ala Gly Gly Asp Phe Gly Phe Gly Ile
            400                 405                 410
Tyr Asn Asp His His Tyr His Leu Gly Tyr Phe Ile Tyr Gly Ile Ala Val Leu
415                 420                 425                 430
Thr Lys Leu Asp Pro Ala Trp Gly Arg Lys Tyr Lys Pro Gln Ala Tyr Ser Ile
        435                 440                 445                 450
Val Gln Asp Phe Leu Asn Leu Asp Thr Lys Leu Asn Ser Asn Tyr Thr Arg Leu

                455                 460                 465
Arg Cys Phe Asp Pro Tyr Val Leu His Ser Trp Ala Gly Gly Leu Thr Glu Phe
    470                 475                 480                 485
Thr Asp Gly Arg Asn Gln Glu Ser Thr Ser Glu Ala Val Ser Ala Tyr Tyr Ser
            490                 495                 500
Ala Ala Leu Met Gly Leu Ala Tyr Gly Asp Ala Pro Leu Val Ala Leu Gly Ser
505                 510                 515                 520
Thr Leu Thr Ala Leu Glu Ile Glu Gly Thr Lys Met Trp Trp His Val Lys Glu
        525                 530                 535                 540
Gly Gly Thr Leu Tyr Glu Lys Glu Phe Thr Gln Glu Asn Arg Val Met Gly Val
                545                 550                 555
Leu Trp Ser Asn Lys Arg Asp Thr Gly Leu Trp Phe Ala Pro Ala Glu Trp Lys
    560                 565                 570                 575
Glu Cys Arg Leu Gly Ile Gln Leu Leu Pro Leu Ala Pro Ile Ser Glu Ala Ile
            580                 585                 590
Phe Ser Asn Val Asp Phe Val Lys Glu Leu Val Glu Trp Thr Leu Pro Ala Leu
595                 600                 605                 610
Asp Arg Glu Gly Gly Val Gly Glu Gly Trp Lys Gly Phe Val Tyr Ala Leu Glu
        615                 620                 625                 630
Gly Val Tyr Asp Asn Glu Ser Ala Leu Gln Lys Ile Arg Asn Leu Lys Gly Phe
                635                 640                 645
Asp Gly Gly Asn Ser Leu Thr Asn Leu Leu Trp Trp Ile His Ser Arg Ser Asp
    650                 655                 660                 665
Glu
667

INFORMATION FOR SEQ ID NO: 2
LENGTH: 2004 base pairs
TYPE: nucleic acid

STRANDEDNESS: double

TOPOLOGY: linear

MOLECULE TYPE: cDNA

ORIGINAL SOURCE:

ORGANISM: Soybean (Glycine max L.)

STRAIN: Green Homer

SEQUENCE DESCRIPTION: SEQ ID NO: 2:

9 18 27 36 45 54

GTT AAC ATC CAA ACC AAT ACA TCT TAC ATC TTC CCT CAA ACA CAA TCC ACT GTT

Val Asn Ile Gln Thr Asn Thr Ser Tyr Ile Phe Pro Gln Thr Gln Ser Thr Val

63 72 81 90 99 108

CTT CCT GAT CCC TCC AAA TTC TTC TCC TCA AAC CTT CTC TCA AGT CCA CTC CCC

Leu Pro Asp Pro Ser Lys Phe Phe Ser Ser Asn Leu Leu Ser Ser Pro Leu Pro

117 126 135 144 153 162

ACA AAC TCT TTC TTC CAA AAC TTT GTC CTA AAA AAT GGT GAC CAA CAA GAA TAC

Thr Asn Ser Phe Phe Gln Asn Phe Val Leu Lys Asn Gly Asp Gln Gln Glu Tyr

171 180 189 198 207 216

ATT CAT CCT TAC CTC ATC AAA TCC TCC AAC TCT TCC CTC TCT CTC TCA TAC CCT

Ile His Pro Tyr Leu Ile Lys Ser Ser Asn Ser Ser Leu Ser Leu Ser Tyr Pro

225 234 243 252 261 270

TCT CGC CAA GCC AGT TCA GCT GTC ATA TTC CAA GTC TTC AAT CCT GAT CTT ACC

Ser Arg Gln Ala Ser Ser Ala Val Ile Phe Gln Val Phe Asn Pro Asp Leu Thr

279 288 297 306 315 324

ATT TCA GCC CCA CAA GGT CCC AAA CAA GGT CCC CCT GGT AAA CAC CTT ATC TCC

Ile Ser Ala Pro Gln Gly Pro Lys Gln Gly Pro Pro Gly Lys His Leu Ile Ser

333 342 351 360 369 378

TCC TAC AGT GAT CTC AGT GTC ACC TTG GAT TTC CCT TCT TCC AAT CTG AGC TTC

Ser Tyr Ser Asp Leu Ser Val Thr Leu Asp Phe Pro Ser Ser Asn Leu Ser Phe

387 396 405 414 423 432

TTC CTT GTT AGG GGA AGC CCC TAT TTG ACT GTG TCT GTG ACT CAA CCA ACT CCT

Phe Leu Val Arg Gly Ser Pro Tyr Leu Thr Val Ser Val Thr Gln Pro Thr Pro

441 450 459 468 477 486

CTT TCA ATT ACC ACC ATC CAT TCC ATT CTC TCA TTC TCT TCA AAT GAC TCC AAC

Leu Ser Ile Thr Thr Ile His Ser Ile Leu Ser Phe Ser Ser Asn Asp Ser Asn

495 504 513 522 531 540

ACC AAG TAC ACC TTT CAG TTC AAC AAT GGT CAA ACA TGG CTT CTT TAT GCT ACC

Thr Lys Tyr Thr Phe Gln Phe Asn Asn Gly Gln Thr Trp Leu Leu Tyr Ala Thr

549 558 567 576 585 594

TCC CCC ATC AAG TTG AAC CAC ACC CTT TCT GAG ATA ACT TCT AAT GCA TTT TCT

Ser Pro Ile Lys Leu Asn His Thr Leu Ser Glu Ile Thr Ser Asn Ala Phe Ser

603 612 621 630 639 648

GGC ATA ATC CGG ATA GCT TTG TTG CCG GAT TCG GAT TCG AAA CAC GAG GCT GTT

Gly Ile Ile Arg Ile Ala Leu Leu Pro Asp Ser Asp Ser Lys His Glu Ala Val

657 666 675 684 693 702

CTT GAC AAG TAT AGT TCT TGT TAC CCC GTG TCA GGT AAA GCT GTG TTC AGA GAA

Leu Asp Lys Tyr Ser Ser Cys Tyr Pro Val Ser Gly Lys Ala Val Phe Arg Glu

711 720 729 738 747 756
CCT TTC TGT GTG GAA TAT AAC TGG GAG AAG AAA GAT TCA GGG GAT TTG CTA CTC
Pro Phe Cys Val Glu Tyr Asn Trp Glu Lys Lys Asp Ser Gly Asp Leu Leu Leu

765 774 783 792 801 810
TTG GCT CAC CCT CTC CAT GTT CAG CTT CTT CGT AAT GGA GAC AAT GAT GTC AAA
Leu Ala His Pro Leu His Val Gln Leu Leu Arg Asn Gly Asp Asn Asp Val Lys

819 828 837 846 855 864
ATT CTT GAA GAT TTA AAG TAT AAA AGC ATT GAT GGG GAT CTT GTT GGT GTT GTC
Ile Leu Glu Asp Leu Lys Tyr Lys Ser Ile Asp Gly Asp Leu Val Gly Val Val

873 882 891 900 909 918
GGG GAT TCA TGG GTT TTG AAA ACA GAT CCT TTG TTT GTA ACA TGG CAT TCA ATC
Gly Asp Ser Trp Val Leu Lys Thr Asp Pro Leu Phe Val Thr Trp His Ser Ile

927 936 945 954 963 972
AAG GGA ATC AAA GAA GAA TCC CAT GAT GAG ATT GTC TCA GCC CTT TCT AAA GAT
Lys Gly Ile Lys Glu Glu Ser His Asp Glu Ile Val Ser Ala Leu Ser Lys Asp

981 990 999 1008 1017 1026
GTT GAG AGC CTA GAT TCA TCA TCA ATA ACT ACA ACA GAG TCA TAT TTT TAT GGG
Val Glu Ser Leu Asp Ser Ser Ser Ile Thr Thr Thr Glu Ser Tyr Phe Tyr Gly

1035 1044 1053 1062 1071 1080
AAG TTG ATT GCA AGG GCT GCA AGG TTG GTA TTG ATT GCT GAG GAG TTG AAC TAC
Lys Leu Ile Ala Arg Ala Ala Arg Leu Val Leu Ile Ala Glu Glu Leu Asn Tyr

1089 1098 1107 1116 1125 1134
CCT GAT GTG ATT CCA AAG GTT AGG AAT TTT TTG AAA GAA ACC ATT GAG CCA TGG
Pro Asp Val Ile Pro Lys Val Arg Asn Phe Leu Lys Glu Thr Ile Glu Pro Trp

1143 1152 1161 1170 1179 1188
TTG GAG GGA ACT TTT AGT GGG AAT GGA TTC CTA CAT GAT GAA AAA TGG GGT GGC
Leu Glu Gly Thr Phe Ser Gly Asn Gly Phe Leu His Asp Glu Lys Trp Gly Gly

1197 1206 1215 1224 1233 1242
ATT ATT ACC CAA AAG GGG TCC ACT GAT GCT GGT GGT GAT TTT GGA TTT GGA ATT
Ile Ile Thr Gln Lys Gly Ser Thr Asp Ala Gly Gly Asp Phe Gly Phe Gly Ile

1251 1260 1269 1278 1287 1296
TAC AAT GAT CAC CAC TAT CAT TTG GGG TAC TTC ATT TAT GGA ATT GCG GTG CTC
Tyr Asn Asp His His Tyr His Leu Gly Tyr Phe Ile Tyr Gly Ile Ala Val Leu

1305 1314 1323 1332 1341 1350
ACT AAG CTT GAT CCA GCA TGG GGT AGG AAG TAC AAG CCT CAA GCC TAT TCA ATA
Thr Lys Leu Asp Pro Ala Trp Gly Arg Lys Tyr Lys Pro Gln Ala Tyr Ser Ile

1359 1368 1377 1386 1395 1404
GTG CAA GAC TTC TTG AAC TTG GAC ACA AAA TTA AAC TCC AAT TAC ACA CGT TTG
Val Gln Asp Phe Leu Asn Leu Asp Thr Lys Leu Asn Ser Asn Tyr Thr Arg Leu

1413 1422 1431 1440 1449 1458
AGG TGT TTT GAC CCT TAT GTG CTT CAC TCT TGG GCT GGA GGG TTA ACT GAG TTC
Arg Cys Phe Asp Pro Tyr Val Leu His Ser Trp Ala Gly Gly Leu Thr Glu Phe

1467 1476 1485 1494 1503 1512

ACA GAT GGA AGG AAT CAA GAG AGC ACA AGT GAG GCT GTG AGT GCA TAT TAT TCT
Thr Asp Gly Arg Asn Gln Glu Ser Thr Ser Glu Ala Val Ser Ala Tyr Tyr Ser

1521 1530 1539 1548 1557 1566
GCT GCT TTG ATG GGA TTA GCA TAT GGT GAT GCA CCT CTT GTT GCA CTT GGA TCA
Ala Ala Leu Met Gly Leu Ala Tyr Gly Asp Ala Pro Leu Val Ala Leu Gly Ser

1575 1584 1593 1602 1611 1620
ACA CTC ACA GCA TTG GAA ATT GAA GGG ACT AAA ATG TGG TGG CAT GTG AAA GAG
Thr Leu Thr Ala Leu Glu Ile Glu Gly Thr Lys Met Trp Trp His Val Lys Glu

1629 1638 1647 1656 1665 1674
GGA GGT ACT TTG TAT GAG AAA GAG TTT ACA CAA GAG AAT AGG GTG ATG GGT GTT
Gly Gly Thr Leu Tyr Glu Lys Glu Phe Thr Gln Glu Asn Arg Val Met Gly Val

1683 1692 1701 1710 1719 1728
CTA TGG TCT AAC AAG AGG GAC ACT GGA CTT TGG TTT GCT CCT GCT GAG TGG AAA
Leu Trp Ser Asn Lys Arg Asp Thr Gly Leu Trp Phe Ala Pro Ala Glu Trp Lys

1737 1746 1755 1764 1773 1782
GAG TGT AGG CTT GGC ATT CAG CTC TTA CCA TTG GCT CCT ATT TCT GAA GCC ATT
Glu Cys Arg Leu Gly Ile Gln Leu Leu Pro Leu Ala Pro Ile Ser Glu Ala Ile

1791 1800 1809 1818 1827 1836
TTC TCC AAT GTT GAC TTT GTA AAG GAG CTT GTG GAG TGG ACT TTG CCT GCT TTG
Phe Ser Asn Val Asp Phe Val Lys Glu Leu Val Glu Trp Thr Leu Pro Ala Leu

1845 1854 1863 1872 1881 1890
GAT AGG GAG GGT GGT GTT GGT GAA GGA TGG AAG GGG TTT GTG TAT GCC CTT GAA

Asp Arg Glu Gly Gly Val Gly Glu Gly Trp Lys Gly Phe Val Tyr Ala Leu Glu

1899 1908 1917 1926 1935 1944

GGG GTT TAT GAC AAT GAA AGT GCA CTG CAG AAG ATA AGA AAC CTG AAA GGT TTT

Gly Val Tyr Asp Asn Glu Ser Ala Leu Gln Lys Ile Arg Asn Leu Lys Gly Phe

1953 1962 1971 1980 1989 1998

GAT GGT GGA AAC TCT TTG ACC AAT CTC TTG TGG TGG ATT CAT AGC AGA AGT GAT

Asp Gly Gly Asn Ser Leu Thr Asn Leu Leu Trp Trp Ile His Ser Arg Ser Asp

2004

GAA TAG

Glu

INFORMATION FOR SEQ ID NO:3

SEQUENCE LENGTH: 347 amino acids

SEQUENCE TYPE: amino acid

TOPOLOGY: linear

MOLECULE SEQUENCE TYPE: peptide

SEQUENCE DESCRIPTION: SEQ ID NO:3:

Met Ala Lys Tyr His Ser Ser Gly Lys Ser Ser Ser Met Thr Ala Ile Ala Phe

1               5                   10                  15

Leu Phe Ile Leu Leu Ile Thr Tyr Thr Gly Thr Thr Asp Ala Gln Ser Gly Val

    20                  25                  30                  35

Cys Tyr Gly Arg Leu Gly Asn Asn Leu Pro Thr Pro Gln Glu Val Val Ala Leu

            40                  45                  50

Tyr Asn Gln Ala Asn Ile Arg Arg Met Arg Ile Tyr Gly Pro Ser Pro Glu Val

55                  60                  65                  70

Leu Glu Ala Leu Arg Gly Ser Asn Ile Glu Leu Leu Leu Asp Ile Pro Asn Asp
        75                  80                  85                  90
Asn Leu Arg Asn Leu Ala Ser Ser Gln Asp Asn Ala Asn Lys Trp Val Gln Asp
                95                  100                 105
Asn Ile Lys Asn Tyr Ala Asn Asn Val Arg Phe Arg Tyr Val Ser Val Gly Asn
    110                 115                 120                 125
Glu Val Lys Pro Glu His Ser Phe Ala Gln Phe Leu Val Pro Ala Leu Glu Asn
            130                 135                 140
Ile Gln Arg Ala Ile Ser Asn Ala Gly Leu Gly Asn Gln Val Lys Val Ser Thr
145                 150                 155                 160
Ala Ile Asp Thr Gly Ala Leu Ala Glu Ser Phe Pro Pro Ser Lys Gly Ser Phe
        165                 170                 175                 180
Lys Ser Asp Tyr Arg Gly Ala Tyr Leu Asp Gly Val Ile Arg Phe Leu Val Asn
                185                 190                 195
Asn Asn Ala Pro Leu Met Val Asn Val Tyr Ser Tyr Phe Ala Tyr Thr Ala Asn
    200                 205                 210                 215
Pro Lys Asp Ile Ser Leu Asp Tyr Ala Leu Phe Arg Ser Pro Ser Val Val Val
            220                 225                 230
Gln Asp Gly Ser Leu Gly Tyr Arg Asn Leu Phe Asp Ala Ser Val Asp Ala Val
235                 240                 245                 250
Tyr Ala Ala Leu Glu Lys Ala Gly Gly Gly Ser Leu Asn Ile Val Val Ser Glu
        255                 260                 265                 270
Ser Gly Trp Pro Ser Ser Gly Gly Thr Ala Thr Ser Leu Asp Asn Ala Arg Thr
                275                 280                 285
Tyr Asn Thr Asn Leu Val Arg Asn Val Lys Gln Gly Thr Pro Lys Arg Pro Gly
    290                 295                 300                 305
Ala Pro Leu Glu Thr Tyr Val Phe Ala Met Phe Asp Glu Asn Gln Lys Gln Pro
            310                 315                 320
Glu Phe Glu Lys Phe Trp Gly Leu Phe Ser Pro Ile Thr Lys Gln Pro Lys Tyr

325                 330                 335                 340

Ser Ile Asn Phe Asn

345 347

INFORMATION FOR SEQ ID NO: 4

SEQUENCE LENGTH: 1044 base pairs

SEQUENCE TYPE: nucleic acid

SEQUENCE DESCRIPTION: SEQ ID NO: 4:

9 18 27 36 45 54

ATG GCT AAG TAT CAT TCA AGT GGG AAA AGC TCT TCC ATG ACT GCT ATA GCC TTC

Met Ala Lys Tyr His Ser Ser Gly Lys Ser Ser Ser Met Thr Ala Ile Ala Phe

63 72 81 90 99 108

CTG TTT ATC CTT CTA ATC ACT TAT ACA GGC ACA ACA GAT GCA CAA TCC GGG GTA

Leu Phe Ile Leu Leu Ile Thr Tyr Thr Gly Thr Thr Asp Ala Gln Ser Gly Val

117 126 135 144 153 162

TGT TAT GGA AGA CTT GGC AAC AAC TTA CCA ACC CCT CAA GAA GTT GTG GCC CTC

Cys Tyr Gly Arg Leu Gly Asn Asn Leu Pro Thr Pro Gln Glu Val Val Ala Leu

171 180 189 198 207 216

TAC AAT CAA GCC AAC ATT CGC AGG ATG CGA ATC TAC GGT CCA AGC CCA GAA GTC

Tyr Asn Gln Ala Asn Ile Arg Arg Met Arg Ile Tyr Gly Pro Ser Pro Glu Val

225 234 243 252 261 270

CTC GAA GCA CTA AGA GGT TCC AAC ATT GAG CTT TTG CTA GAC ATT CCA AAT GAC

Leu Glu Ala Leu Arg Gly Ser Asn Ile Glu Leu Leu Leu Asp Ile Pro Asn Asp

279 288 297 306 315 324

AAC CTC AGA AAC CTA GCA TCT AGC CAA GAC AAT GCA AAC AAA TGG GTG CAA GAC

Asn Leu Arg Asn Leu Ala Ser Ser Gln Asp Asn Ala Asn Lys Trp Val Gln Asp

333 342 351 360 369 378

AAC ATC AAA AAC TAT GCC AAC AAT GTC AGA TTC AGA TAC GTT TCA GTG GGA AAT

Asn Ile Lys Asn Tyr Ala Asn Asn Val Arg Phe Arg Tyr Val Ser Val Gly Asn

387 396 405 414 423 432

GAA GTG AAA CCC GAA CAC TCA TTT GCA CAA TTT CTA GTG CCT GCA TTG GAA AAC

Glu Val Lys Pro Glu His Ser Phe Ala Gln Phe Leu Val Pro Ala Leu Glu Asn

441 450 459 468 477 486

ATT CAG AGG GCC ATT TCT AAT GCT GGC CTT GGA AAC CAA GTA AAA GTT TCC ACT

Ile Gln Arg Ala Ile Ser Asn Ala Gly Leu Gly Asn Gln Val Lys Val Ser Thr

495 504 513 522 531 540

GCC ATT GAT ACT GGT GCC TTG GCA GAA TCA TTC CCA CCA TCA AAG GGT TCC TTC

Ala Ile Asp Thr Gly Ala Leu Ala Glu Ser Phe Pro Pro Ser Lys Gly Ser Phe

549 558 567 576 585 594

AAA TCT GAT TAT AGA GGA GCA TAT CTT GAT GGT GTC ATC AGA TTT CTA GTG AAC

Lys Ser Asp Tyr Arg Gly Ala Tyr Leu Asp Gly Val Ile Arg Phe Leu Val Asn

603 612 621 630 639 648

AAT AAT GCC CCA TTA ATG GTT AAT GTG TAC TCT TAC TTC GCT TAC ACT GCA AAC

Asn Asn Ala Pro Leu Met Val Asn Val Tyr Ser Tyr Phe Ala Tyr Thr Ala Asn

657 666 675 684 693 702

CCT AAG GAC ATT AGT CTT GAC TAT GCA CTT TTT AGG TCT CCT TCG GTG GTA GTG

Pro Lys Asp Ile Ser Leu Asp Tyr Ala Leu Phe Arg Ser Pro Ser Val Val Val

711 720 729 738 747 756

CAA GAT GGT TCA CTT GGT TAC CGT AAC CTC TTT GAT GCT TCG GTT GAT GCT GTT

Gln Asp Gly Ser Leu Gly Tyr Arg Asn Leu Phe Asp Ala Ser Val Asp Ala Val

765 774 783 792 801 810

TAT GCT GCA TTG GAG AAA GCA GGA GGA GGG TCA TTG AAC ATA GTT GTG TCT GAG

Tyr Ala Ala Leu Glu Lys Ala Gly Gly Gly Ser Leu Asn Ile Val Val Ser Glu

819 828 837 846 855 864

AGT GGA TGG CCT TCT TCT GGT GGA ACT GCA ACT TCA CTT GAT AAT GCA AGA ACT

Ser Gly Trp Pro Ser Ser Gly Gly Thr Ala Thr Ser Leu Asp Asn Ala Arg Thr

873 882 891 900 909 918

TAC AAC ACA AAC TTG GTT CGG AAT GTG AAG CAA GGA ACC CCT AAA AGG CCT GGT
Tyr Asn Thr Asn Leu Val Arg Asn Val Lys Gln Gly Thr Pro Lys Arg Pro Gly
927 936 945 954 963 972
GCA CCC CTT GAA ACT TAT GTG TTT GCC ATG TTT GAT GAA AAT CAG AAG CAG CCA
Ala Pro Leu Glu Thr Tyr Val Phe Ala Met Phe Asp Glu Asn Gln Lys Gln Pro
981 990 999 1008 1017 1026
GAG TTT GAA AAA TTT TGG GGG CTC TTT TCT CCT ATA ACT AAG CAG CCC AAA TAC
Glu Phe Glu Lys Phe Trp Gly Leu Phe Ser Pro Ile Thr Lys Gln Pro Lys Tyr
1035 1044
TCG ATT AAT TTC AAT TAA
Ser Ile Asn Phe Asn

INFORMATION FOR SEQ ID NO:5
SEQUENCE LENGTH: 13 amino acids
SEQUENCE TYPE: amino acid
TOPOLOGY: linear
MOLECULE SEQUENCE TYPE: peptide
SEQUENCE DESCRIPTION: SEQ ID NO:5:
Val Asn Ile Gln Thr Asn Thr Ser Asn Ile Ser Pro Gln
1 5 10 13

INFORMATION FOR SEQ ID NO:6
SEQUENCE LENGTH: 14 amino acids
SEQUENCE TYPE: amino acid
TOPOLOGY: linear
MOLECULE SEQUENCE TYPE: peptide
SEQUENCE DESCRIPTION: SEQ ID NO:6:

Lys Ser Ile Asp Gly Asp Leu Val Gly Val Val Gly Asp Ser
1 5 10 14

INFORMATION FOR SEQ ID NO:7
SEQUENCE LENGTH: 17 amino acids
SEQUENCE TYPE: amino acid
TOPOLOGY: linear
MOLECULE SEQUENCE TYPE: peptide
SEQUENCE DESCRIPTION: SEQ ID NO:7:
Lys Tyr Lys Pro Gln Ala Tyr Ser Ile Val Gln Asp Phe Leu Asn Leu Asp
1 5 10 15 17

INFORMATION FOR SEQ ID NO:8
SEQUENCE LENGTH: 13 amino acids
SEQUENCE TYPE: amino acid
TOPOLOGY: linear
MOLECULE SEQUENCE TYPE: peptide
SEQUENCE DESCRIPTION: SEQ ID NO:8:
Lys Thr Asp Pro Leu Phe Val Thr Trp His Ser Ile Lys
1 5 10 13

INFORMATION FOR SEQ ID NO: 9
SEQUENCE LENGTH: 17 base pairs
SEQUENCE TYPE: nucleic acid
STRANDEDNESS: single
TOPOLOGY: linear

MOLECULE SEQUENCE TYPE: Other nucleic acid, synthetic DNA
SEQUENCE DESCRIPTION: SEQ ID NO: 9:
AARAGYATHG AYGGNGA 17

INFORMATION FOR SEQ ID NO: 10
SEQUENCE LENGTH: 13 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 10:
WRTCNCCNAC NAC 13

INFORMATION FOR SEQ ID NO: 11
SEQUENCE LENGTH: 17 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 11:
GTNAAYAARA TNCARAC 17

INFORMATION FOR SEQ ID NO: 12
SEQUENCE LENGTH: 17 base pairs
SEQUENCE TYPE: nucleic acid
STRANDEDNESS: single

TOPOLOGY: linear
MOLECULE SEQUENCE TYPE: Other nucleic acid, synthetic DNA
SEQUENCE DESCRIPTION: SEQ ID NO: 12:
ARRTTNAGRA ARTCYTC 17

INFORMATION FOR SEQ ID NO: 13
SEQUENCE LENGTH: 24 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 13:
AAGTAYAAGC CRCAAGCCTA TTCA 24

INFORMATION FOR SEQ ID NO: 14
SEQUENCE LENGTH: 17 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 14:
ATCGCCRACA ACMCCAA 17

INFORMATION FOR SEQ ID NO: 15
SEQUENCE LENGTH: 54 base pairs
SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 15:

GGAATTCGAG CTCGGTACCC GGGGGATCCT CTAGAGTCGA CCTGCAGGCA TGCA

INFORMATION FOR SEQ ID NO: 16

SEQUENCE LENGTH: 58 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 16:

CCTTAAGCTC GAGCCATGGG CCCCCTAGGA GATCTCAGCT GGACGTCCGT ACGTTCGA

INFORMATION FOR SEQ ID NO: 17

SEQUENCE LENGTH: 26 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 17:

ATGGATCCAT GGTTAACATC CAAACC 26

INFORMATION FOR SEQ ID NO: 18

SEQUENCE LENGTH: 26 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 18:

ATGGATCCGA ATATAACTGG GAGAAG 26

INFORMATION FOR SEQ ID NO: 19

SEQUENCE LENGTH: 26 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 19:

ATGGATCCCC AGCATGGGGT AGGAAG 26

INFORMATION FOR SEQ ID NO: 20

SEQUENCE LENGTH: 28 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 20:

TAGTCGACTA CTTCTCCCAG TTATATTC 28

INFORMATION FOR SEQ ID NO: 21

SEQUENCE LENGTH: 28 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 21:
TAGTCGACTA CTTCCTACCC CATGCTGG 28

INFORMATION FOR SEQ ID NO: 22
SEQUENCE LENGTH: 28 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 22:
TAGTCGACTA TTCATCACTT CTGCTATG 28

INFORMATION FOR SEQ ID NO: 23
SEQUENCE LENGTH: 26 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 23:
ATGGATCCGC CCCACAAGGT CCCAAA 26

INFORMATION FOR SEQ ID NO: 24
SEQUENCE LENGTH: 26 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 24:
ATGGATCCAA TGACTCCAAC ACCAAG 26

INFORMATION FOR SEQ ID NO: 25
SEQUENCE LENGTH: 26 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 25:
ATGGATCCGA ATATAACTGG GAGAAG 26

INFORMATION FOR SEQ ID NO: 26
SEQUENCE LENGTH: 28 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 26:
TAGTCGACTA CTTCCTACCC CATGCTGG 28

INFORMATION FOR SEQ ID NO: 27

SEQUENCE LENGTH: 34 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 27:

CTAGAGGATC CGGTACCCCC GGGGTCGACG AGCT 34

INFORMATION FOR SEQ ID NO: 28

SEQUENCE LENGTH: 26 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 28:

CGTCGACCCC GGGGGTACCG GATCCT 26

INFORMATION FOR SEQ ID NO: 29

SEQUENCE LENGTH: 20 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 29:

CACCTTCAGC AACAATGGTT 20

INFORMATION FOR SEQ ID NO: 30
SEQUENCE LENGTH: 20 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 30:
CTATTCATCA CTTCTGCTAT 20

INFORMATION FOR SEQ ID NO: 31
SEQUENCE LENGTH: 24 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 31:
CAAATGTTGT GGTGAGGGAT GGCC 24

INFORMATION FOR SEQ ID NO: 32
SEQUENCE LENGTH: 24 base pairs
SEQUENCE TYPE: nucleic acid
STRANDEDNESS: single
TOPOLOGY: linear
MOLECULE SEQUENCE TYPE: Other nucleic acid, synthetic DNA
SEQUENCE DESCRIPTION: SEQ ID NO: 32:

AAATGTTTCT CTATCTCAGG ACTC 24

INFORMATION FOR SEQ ID NO: 33

SEQUENCE LENGTH: 996 base pairs

SEQUENCE TYPE: nucleic acid

STRANDEDNESS: double

TOPOLOGY: linear

MOLECULE SEQUENCE TYPE: cDNA

ORIGINAL SOURCE:

ORGANISM: Kidney bean (Phaseolus vulgaris L.)

STRAIN: Hirasaya Fancy Saitou

SEQUENCE DESCRIPTION: SEQ ID NO: 33:

9 18 27 36 45 54

ATG TCT GCC TTA TTG CTG CTT CTT GGA GTA TTA TCT TCC ACT GGA GTA CTG CTT

Met Ser Ala Leu Leu Leu Leu Leu Gly Val Leu Ser Ser Thr Gly Val Leu Leu

63 72 81 90 99 108

ACT GGG GTA GAA TCT GTG GGT GTG TGT TAT GGA GGA AAT GGA AAC AAT CTA CCA

Thr Gly Val Glu Ser Val Gly Val Cys Tyr Gly Gly Asn Gly Asn Asn Leu Pro

117 126 135 144 153 162

ACA AAG CAA GCA GTG GTG AAT CTC TAC AAA TCA AAC GGA ATT GGC AAA ATC CGT

Thr Lys Gln Ala Val Val Asn Leu Tyr Lys Ser Asn Gly Ile Gly Lys Ile Arg

171 180 189 198 207 216

TTA TAC TAT CCA GAT GAA GGT GCC CTT CAA GCC CTC AGA GGT TCA AAC ATA GAA

Leu Tyr Tyr Pro Asp Glu Gly Ala Leu Gln Ala Leu Arg Gly Ser Asn Ile Glu

225 234 243 252 261 270

GTG ATA CTT GCT GTT CCT AAT GAT CAA CTT CAA TCT GTC TCC AAC AAT GGA AGT

Val Ile Leu Ala Val Pro Asn Asp Gln Leu Gln Ser Val Ser Asn Asn Gly Ser

279 288 297 306 315 324

GCA ACA AAT TGG GTC AAC AAT TAC GTG AAA CCC TAT GCA GGA AAC GTG AAA TTG
Ala Thr Asn Trp Val Asn Asn Tyr Val Lys Pro Tyr Ala Gly Asn Val Lys Leu
333 342 351 360 369 378
AAG TAC ATT GCA GTT GGC AAC GAA GTT CAC CCT GGT GAT GCT CTA GCA GGC TCA
Lys Tyr Ile Ala Val Gly Asn Glu Val His Pro Gly Asp Ala Leu Ala Gly Ser
387 396 405 414 423 432
GTT CTT CCA GCA CTT CAA AGC ATT CAG AAC GCA ATT TCT GCA GCA AAT TTG CAA
Val Leu Pro Ala Leu Gln Ser Ile Gln Asn Ala Ile Ser Ala Ala Asn Leu Gln
441 450 459 468 477 486
CGC CAA ATC AAA GTC TCC ACA GCA ATA GAC ACC ACT CTA CTG GGC AAC TCT TAC
Arg Gln Ile Lys Val Ser Thr Ala Ile Asp Thr Thr Leu Leu Gly Asn Ser Tyr
495 504 513 522 531 540
CCA CCA AAA GAT GGC GTT TTC AGC AAC AGT GCA AGT TCA TAC ATA ACT CCA ATC
Pro Pro Lys Asp Gly Val Phe Ser Asn Ser Ala Ser Ser Tyr Ile Thr Pro Ile
549 558 567 576 585 594
ATA AAC TTT TTA GCC AAA AAC GGT GCC CCA CTT CTT GCA AAC GTG TAC CCT TAC
Ile Asn Phe Leu Ala Lys Asn Gly Ala Pro Leu Leu Ala Asn Val Tyr Pro Tyr
603 612 621 630 639 648
TTC GCC TAC GTT AAC AAT CAA CAA AAC ATT GGT CTT GAT TAT GCC TTG TTT ACC
Phe Ala Tyr Val Asn Asn Gln Gln Asn Ile Gly Leu Asp Tyr Ala Leu Phe Thr
657 666 675 684 693 702
AAA CAA GGC AAC AAC GAA GTT GGG TAC CAA AAC CTG TTT GAT GCA TTG GTG GAT
Lys Gln Gly Asn Asn Glu Val Gly Tyr Gln Asn Leu Phe Asp Ala Leu Val Asp
711 720 729 738 747 756
TCT CTG TAC GCA GCT CTT GAG AAA GTG GGA GCA TCA AAT GTG AAG GTT GTT GTG
Ser Leu Tyr Ala Ala Leu Glu Lys Val Gly Ala Ser Asn Val Lys Val Val Val
765 774 783 792 801 810
TCT GAG AGT GGG TGG CCA TCA CAA GGT GGA GTT GGA GCC ACT GTT CAA AAC GCA
Ser Glu Ser Gly Trp Pro Ser Gln Gly Gly Val Gly Ala Thr Val Gln Asn Ala

819 828 837 846 855 864
GGA ACG TAT TAC AGG AAT TTG ATC AAA CAT GTT AAG GGT GGC ACC CCA AAG AGG
Gly Thr Tyr Tyr Arg Asn Leu Ile Lys His Val Lys Gly Gly Thr Pro Lys Arg
873 882 891 900 909 918
CCT AAT GGA CCC ATA GAG ACT TAC CTC TTT GCC ATG TTT GAT GAA AAC CAG AAG
Pro Asn Gly Pro Ile Glu Thr Tyr Leu Phe Ala Met Phe Asp Glu Asn Gln Lys
927 936 945 954 963 972
GGT GGT GCA GAA ACT GAG AAA CAC TTT GGT CTC TTC AGG CCT GAT AAA TCA CCA
Gly Gly Ala Glu Thr Glu Lys His Phe Gly Leu Phe Arg Pro Asp Lys Ser Pro
981 990 996
AAA TAC CAA CTC AGT TTC AAT TGA
Lys Tyr Gln Leu Ser Phe Asn***

INFORMATION FOR SEQ ID NO:34
SEQUENCE LENGTH: 331 amino acids
SEQUENCE TYPE: amino acid
TOPOLOGY: linear
MOLECULE SEQUENCE TYPE: peptide
SEQUENCE DESCRIPTION: SEQ ID NO:34:
Met Ser Ala Leu Leu Leu Leu Leu Gly Val Leu Ser Ser Thr Gly Val Leu Leu
1               5                   10                  15
Thr Gly Val Glu Ser Val Gly Val Cys Tyr Gly Gly Asn Gly Asn Asn Leu Pro
    20                  25                  30                  35
Thr Lys Gln Ala Val Val Asn Leu Tyr Lys Ser Asn Gly Ile Gly Lys Ile Arg
            40                  45                  50
Leu Tyr Tyr Pro Asp Glu Gly Ala Leu Gln Ala Leu Arg Gly Ser Asn Ile Glu
55                  60                  65                  70
Val Ile Leu Ala Val Pro Asn Asp Gln Leu Gln Ser Val Ser Asn Asn Gly Ser

75                  80                  85                  90
Ala Thr Asn Trp Val Asn Asn Tyr Val Lys Pro Tyr Ala Gly Asn Val Lys Leu
                95                  100                 105
Lys Tyr Ile Ala Val Gly Asn Glu Val His Pro Gly Asp Ala Leu Ala Gly Ser
    110                 115                 120                 125
Val Leu Pro Ala Leu Gln Ser Ile Gln Asn Ala Ile Ser Ala Ala Asn Leu Gln
            130                 135                 140
Arg Gln Ile Lys Val Ser Thr Ala Ile Asp Thr Thr Leu Leu Gly Asn Ser Tyr
145                 150                 155                 160
Pro Pro Lys Asp Gly Val Phe Ser Asn Ser Ala Ser Ser Tyr Ile Thr Pro Ile
        165                 170                 175                 180
Ile Asn Phe Leu Ala Lys Asn Gly Ala Pro Leu Leu Ala Asn Val Tyr Pro Tyr
                185                 190                 195
Phe Ala Tyr Val Asn Asn Gln Gln Asn Ile Gly Leu Asp Tyr Ala Leu Phe Thr
    200                 205                 210                 215
Lys Gln Gly Asn Asn Glu Val Gly Tyr Gln Asn Leu Phe Asp Ala Leu Val Asp
            220                 225                 230
Ser Leu Tyr Ala Ala Leu Glu Lys Val Gly Ala Ser Asn Val Lys Val Val Val
235                 240                 245                 250
Ser Glu Ser Gly Trp Pro Ser Gln Gly Gly Val Gly Ala Thr Val Gln Asn Ala
        255                 260                 265                 270
Gly Thr Tyr Tyr Arg Asn Leu Ile Lys His Val Lys Gly Gly Thr Pro Lys Arg
                275                 280                 285
Pro Asn Gly Pro Ile Glu Thr Tyr Leu Phe Ala Met Phe Asp Glu Asn Gln Lys
    290                 295                 300                 305
Gly Gly Ala Glu Thr Glu Lys His Phe Gly Leu Phe Arg Pro Asp Lys Ser Pro
            310                 315                 320
Lys Tyr Gln Leu Ser Phe Asn***
325                 330

INFORMATION FOR SEQ ID NO: 35

SEQUENCE LENGTH: 29 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 35:

GGAATTCCGA ATCTGTGGGT GTGTGTTAT 29

INFORMATION FOR SEQ ID NO: 36

SEQUENCE LENGTH: 25 base pairs

SEQUENCE TYPE: nucleic acid

STRANDEDNESS: single

TOPOLOGY: linear

MOLECULE SEQUENCE TYPE: Other nucleic acid, synthetic DNA

SEQUENCE DESCRIPTION: SEQ ID NO: 36:

GGAAACAGCT ATGACCATGA TTAGC 25

## Claims

1. A method for creating a plant having resistance to pathogenic fungi, comprising incorporating a DNA sequence coding for a glucan elicitor receptor into a chromosome of a plant and expressing the glucan elicitor receptor in the plant.

2. The method of claim 1, wherein the DNA sequence coding for a glucan elicitor receptor comprises a nucleotide sequence coding for the amino acid sequence substantially shown in SEQ ID NO: 1.

3. The method of claim 1, wherein the DNA sequence coding for a glucan elicitor receptor comprises the nucleotide sequence substantially shown in SEQ ID NO: 2.

4. The method of claim 1, wherein the DNA sequence coding for a glucan elicitor receptor comprises a nucleotide sequence incorporated into plasmid pER23-1.

5. The method of claim 1, further comprising incorporating a DNA sequence coding for a glucanase into a chromosome of the plant and expressing the glucanase in the plant.

6. The method of claim 5, wherein the DNA sequence coding for a glucanase comprises a nucleotide sequence coding for the amino acid sequence substantially shown in SEQ ID NO: 3 or 34.

7. The method of claim 5, wherein the DNA sequence coding for a glucanase comprises the nucleotide sequence substantially shown in SEQ ID NO: 4 or 33.

8. The method of claim 1, wherein the DNA sequence coding for a glucan elicitor receptor is transferred into Agrobacterium and then the Agrobacterium is allowed to infect a plant to thereby incorporate said glucan elicitor receptor encoding DNA sequence into a chromosome of the plant.

9. The method of claim 1, wherein the DNA sequence coding for a glucan elicitor receptor is transferred into a plant material selected from the group consisting of leaves, stems, roots, tubers, protoplasts, calluses, seed embryos, shoot primordia and pollen.

10. The method of claim 9, wherein the DNA sequence coding for a glucan elicitor receptor is transferred into a plant material selected from the group consisting of leaves, stems, roots, tubers, protoplasts, calluses, seed embryos, shoot primordia and pollen, and then a plant is regenerated from said plant material.

11. The method of claim 1, wherein the pathogenic fungi contain glucan in their cell wall components.

12. The method of claim 1, wherein the pathogenic fungi belong to the genus Phytophthora or Rhizoctonia.

13. The method of claim 1, wherein the plant is susceptible to infection with pathogenic fungi containing glucan in their cell wall components.

14. The method of claim 1, wherein the plant is a solanaceous plant or a leguminous plant.

15. A plant having resistance to pathogenic fungi, said plant having a DNA sequence coding for a glucan elicitor receptor transferred thereinto and expressing the glucan elicitor receptor, or the progeny of said plant.

16. The plant or its progeny of claim 15, wherein the DNA sequence coding for a glucan elicitor receptor comprises a nucleotide sequence coding for the amino acid sequence substantially shown in SEQ ID NO: 1.

17. The plant or its progeny of claim 15, wherein the DNA sequence coding for a glucan elicitor receptor comprises the nucleotide sequence substantially shown in SEQ ID NO: 2.

18. The plant or its progeny of claim 15, wherein the DNA sequence coding for a glucan elicitor receptor comprises a nucleotide sequence incorporated into plasmid pER23-1.

19. The plant or its progeny of claim 15, wherein the plant further has a DNA sequence coding for a glucanase transferred thereinto and expresses the glucanase.

20. The plant or its progeny of claim 19, wherein the DNA sequence coding for a glucanase comprises a nucleotide sequence coding for the amino acid sequence substantially shown in SEQ ID NO: 3 or 34.

21. The plant or its progeny of claim 19, wherein the DNA sequence coding for a glucanase comprises the nucleotide sequence substantially shown in SEQ ID NO: 4 or 33.

22. The plant or its progeny of claim 15, wherein the pathogenic fungi contain glucan in their cell wall components.

23. The plant or its progeny of claim 15, wherein the pathogenic fungi belong to the genus Phytophthora or Rhizoctonia.

24. The plant or its progeny of claim 15, wherein the plant is susceptible to infection with pathogenic fungi containing glucan in their cell wall components.

25. The plant or its progeny of claim 15, wherein the plant is a solanaceous plant or a leguminous plant.

26. Use of a DNA sequence coding for a glucan elicitor receptor for the purpose of conferring on a plant resistance to pathogenic fungi or for the purpose of enhancing the resistance of a plant to pathogenic fungi.

27. Use of a DNA sequence coding for a glucan elicitor receptor for the purpose of creating a plant having resistance to pathogenic fungi.
